# EUROPEAN PATENT SPECIFICATION

(11) **EP 3 256 124 B1**
(45) Date of publication and mention of the grant of the patent: **23.12.2020**
(21) Application number: 15882259.3
(22) Date of filing: 22.09.2015
(51) Int. Cl.: A61K 31/4178, A61K 31/194, A61K 31/198, A61K 45/06, A61K 31/395, A61P 1/16, A61P 1/04, A61P 29/00

(54) **COMPOSITION COMPRISING CENICRIVIROC AND FUMARIC ACID FOR USE IN THE TREATMENT OF ACUTE LIVER INJURY OR PERITONITIS**
ZUSAMMENSETZUNG ENTHALTEND CENICRIVIROC UND FUMARSÄURE ZUR BEHANDLUNG VON AKUTEN LEBERVERLETZUNGEN ODER PERITONITIS
COMPOSITION COMPRENANT LE CÉNICRIVIROC ET DE L'ACIDE FUMARIQUE DESTINÉE À ÊTRE UTILISÉE DANS LE TRAITEMENT DE LÉSIONS HÉPATIQUES AIGUËS OU DE LA PÉRITONITE

(30) Priority: 10.02.2015 US 201562114304 P
(43) Date of publication of application: 20.12.2017
(73) Proprietor: Tobira Therapeutics, Inc., South San Francisco CA 94080 (US)
(72) Inventor: LEFEBVRE, Eric, South San Francisco, CA 94080 (US)
(74) Representative: J A Kemp LLP
(86) International application number: PCT/US2015/051467
(87) International publication number: WO 2016/130179

(56) References cited:
- WO-A1-2013/000922
- WO-A1-2014/186581
- GB-A- 2 249 027
- S. Friedman: "Significant Anti-Fibrotic Activity of Cenicriviroc, A Dual. CCR2/CCR5 Antagonist, in a Rat Model of Thioacetamide-Induced Liver Fibrosis and Cirrhosis", AASLD Liver Learning, 4 November 2013 (2013-11-04), pages 1-2, XP055499943, Retrieved from the Internet: URL:http://liverlearning.aasld.org/aasld/2 013/thelivermeeting/42765/scott.l.friedman .significant.anti-fibrotic.activity.of.cen icriviroc.a.dual.html [retrieved on 2018-08-15]
- Anonymous: "Tobira Therapeutics Presents Data Supporting Anti-Fibrotic Activity of Cenicriviroc in Liver Disease Models at The Liver Meeting 2013 | Business Wire", AASLD ANNUAL MEETING, 1 January 2013 (2013-01-01), pages 1-2, XP055417275, Retrieved from the Internet: URL:http://www.businesswire.com/news/home/ 20131104005506/en/Tobira-Therapeutics-Pres ents-Data-Supporting-Anti-Fibrotic-Activit y [retrieved on 2017-10-19]
- PETER NORMAN: "A dual CCR2/CCR5 chemokine antagonist, BMS-813160? : Evaluation of WO2011046916", EXPERT OPINION ON THERAPEUTIC PATENTS, vol. 21, no. 12, 22 September 2011 (2011-09-22), pages 1919-1924, XP55446959, ISSN: 1354-3776, DOI: 10.1517/13543776.2011.622750
- HIROTAKE TOKUYAMA ET AL: "The simultaneous blockade of chemokine receptors CCR2, CCR5 and CXCR3 by a non-peptide chemokine receptor antagonist protects mice from dextran sodium sulfate-mediated colitis", INTERNATIONAL IMMUNOLOGY, vol. 17, no. 8, 1 August 2005 (2005-08-01) , pages 1023-1034, XP055500146, ISSN: 0953-8178, DOI: 10.1093/intimm/dxh284
- JUNKER A ET AL: "Selective and dual targeting of CCR2 and CCR5 receptors: A current overview", TOPICS IN MEDICINAL CHEMISTRY, SPRINGER, DE, vol. 14, 5 March 2014 (2014-03-05), pages 187-242, XP009507464, ISSN: 1862-2461, DOI: 10.1007/7355_2014_40
- MARK M. MENNING ET AL: "Fumaric Acid Microenvironment Tablet Formulation and Process Development for Crystalline Cenicriviroc Mesylate, a BCS IV Compound", MOLECULAR PHARMACEUTICS, vol. 10, no. 11, 4 November 2013 (2013-11-04), pages 4005-4015, XP055295392, US ISSN: 1543-8384, DOI: 10.1021/mp400286s
- ACUTE LIVER FAILURE STUDY GROUP ET AL: "Intravenous N-Acetylcysteine Improves Transplant-Free Survival in Early Stage Non-Acetaminophen Acute Liver Failure", GASTROENTEROLOGY, W.B. SAUNDERS CO, US, vol. 137, no. 3, 1 September 2009 (2009-09-01), pages 856-864.e1, XP026792978, ISSN: 0016-5085 [retrieved on 2009-06-12]
- CROWLEY B: "Antibiotic therapy and peritonitis", LANCET, ELSEVIER, AMSTERDAM, NL, vol. 357, no. 9253, 3 February 2001 (2001-02-03), page 396, XP004799445, ISSN: 0140-6736, DOI: 10.1016/S0140-6736(05)71532-4
- O Krenkel ET AL: "ORAL PRESENTATIONS O033 CCR2+ INFILTRATING MONOCYTES PROMOTE ACETAMINOPHEN-INDUCED ACUTE LIVER INJURY - THERAPEUTIC IMPLICATIONS OF INHIBITING CCR2 AND CCL2", Journal of Hepatology, 1 April 2015 (2015-04-01), page S206, XP055499948, Retrieved from the Internet: URL:https://www.journal-of-hepatology.eu/a rticle/S0168-8278(15)30041-6/pdf [retrieved on 2018-08-15]
- 'Tobira therapeutics presents data supporting anti-fibrotic activity of cenicriviroc in liver disease models at the liver meeting 2013' AASLD ANNUAL MEETING vol. 2013, 2013, pages 1 - 3, XP055417275 Retrieved from the Internet: <URL:http://www.businesswire.com/news/home/ 20131104005506/en/Tobira- Therapeutics-Presents-Data-Supporting-Anti- Fibrotic-Activity>
- KLIBANOV, 0. M. ET AL.: 'Cenicriviroc, an orally active CCR5 antagonist for the potential treatment of HIV infection' CURRENT OPINION IN INVESTIGATIONAL DRUGS vol. 11, no. 8, 2010, pages 940 - 950, XP055372828
- MENNING, M. M. ET AL.: 'Fumaric acid microenvironment tablet formulation and process development for crystalline cenicriviroc mesylate, a BCS IV compound' MOLECULAR PHARMACEUTICS vol. 10, 2013, pages 4005 - 4015, XP055295392
- O'GRADY JOHN: "Attempting to predict the unpredictable in acute liver injury.", JOURNAL OF HEPATOLOGY JAN 2005, vol. 42, no. 1, January 2005 (2005-01), pages 5-6, ISSN: 0168-8278
- Yedidya Saiman ET AL: "The Role of Chemokines in Acute Liver Injury", Frontiers in Physiology, vol. 3, 1 January 2012 (2012-01-01), XP055306679, DOI: 10.3389/fphys.2012.00213
- BABA M ET AL: "A SMALL-MOLECULAR, NONPEPTIDE CCR5 ANTAGONIST WITH HIGHLY POTENT AND SELECTIVE ANTI-HIV-1 ACTIVITY", PROCEEDINGS OF THE NATIONAL ACADEMY OF SCIENCES, NATIONAL ACADEMY OF SCIENCES, US, vol. 96, 1 May 1999 (1999-05-01), pages 5698-5703, XP000882742, ISSN: 0027-8424, DOI: 10.1073/PNAS.96.10.5698

## Description

### FIELD

The present disclosure relates to a pharmaceutical composition comprising cenicriviroc a salt or solvate thereof, and such compositions for use in the treatment of inflammation and connective tissue diseases and disorders, especially peritonitis, and liver injury, as defined in the claims.

### BACKGROUND

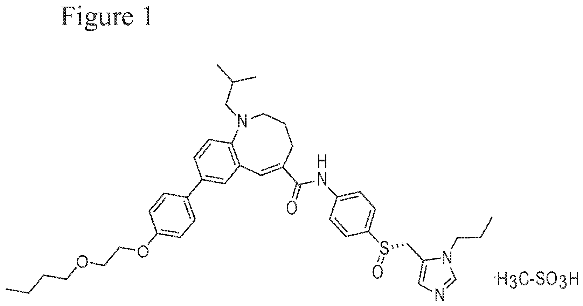

Cenicriviroc (also known as CVC) is the common name of (S,E)-8-(4-(2-Butoxyethoxy)phenyl)-1-(2-methylpropyl)-N-(4-(((1-propyl-1H-imidazol-5-yl)methyl)sulfinyl)phenyl)-1,2,3,4-tetrahydrobenzo[b]azocine-5-carboxamide. The chemical structure of cenicriviroc mesylate appears in Figure 1. Cenicriviroc binds to and inhibits the activity of the C-C chemokine receptor type 2 (CCR2) and C-C chemokine receptor type 5 (CCR5) receptors (24). These receptors not only play a role in entry of viruses such as Human Immunodeficiency Virus (HIV) into the cell, but also are important for the recruitment of immune cells to sites of injury. Inhibition of this receptor's activity may have an anti-inflammatory effect. More recently, the role that inflammation plays in the development of fibrosis has been examined [30]. It has been shown that C-C chemokine receptor type 2 (CCR2) and CCR5 may play a role in promoting hepatic fibrosis [3, 4, 5, 31 32].
Friedman S., AASLD LiverLearning, 4 November 2013, 42765 reports that in a rat model of thioacetamide-induced liver fibrosis and cirrhosis, cenicriviroc showed significant anti-fibrotic activity. Business Wire reports on the AASLD annual meeting 2003 and indicates on 4 November 2013 that positive results were achieved in an evaluation of cenicriviroc into animal models of liver fibrosis. The first experiment evaluated a diet-induced mouse model of non-alcoholic steatohepatitis. The second experiment evaluated a thioacetamide acid induced rat model of liver fibrosis and cirrhosis. CVC treatment was reported to be associated with an anti-fibrotic effect in both models.
Tokuyama et al, International Immunology, 17, 8, 1023-1034 (2005) reports that the simultaneous blockage of chemokine receptors CCR2, CCR5 and CXCR3 by a non-peptide chemokine receptor 121627680 v2 antagonist protects mice from dextran sodium sulphate-mediated colitis. The investigation evaluates the effect of TAK-779 (N,N-dimethyl-N-[4-[[[2-(4-methylphenyl)-6,7-dihydro-5H-benzocyclohepten-8-yl]carbonyl]amino]benzyl]-tetrahydro-2H-pyran-4-aminiuim chloride) as the non-peptide chemokine receptor antagonist.

### SUMMARY OF THE INVENTION

The present invention provides a pharmaceutical composition comprising cenicriviroc or a salt or solvate thereof and fumaric acid for use in the treatment of acute liver injury in a subject in need thereof, wherein the acute liver injury is acetaminophen-induced, and/or toxin-induced liver injury and wherein the pharmaceutical composition is administered to a subject in need thereof in a therapeutically effective amount once per day or twice per day.

The invention also provides a pharmaceutical composition comprising cenicriviroc or a salt or solvate thereof and fumaric acid for use in the treatment of peritonitis in a subject in need thereof;
wherein the pharmaceutical composition is administered to the subject in a therapeutically effective amount twice per day. In one embodiment, the peritonitis is infected or non-infected. In another embodiment, the peritonitis is associated with a perforation of the gastrointestinal tract, leakage of fluid into the peritoneum, or a foreign body.

Preferably, the pharmaceutical composition for use in accordance with the present invention is coadministered with one or more additional active agents or treatments;
wherein the one or more additional active agents or treatments are selected from the group consisting of one or more antibiotic, glucocorticoid, corticosteroid, Pentoxifylline, phosphodiesterase inhibitor, anti-TNFa agent, anti-oxidants and n-acetylcysteine (acetylcysteine; NAC). In one embodiment, the one or more antibiotics is selected from the group consisting of penicillins, cephalosporins, macrolides, fluoroquinolones, sulfonamides, tetracyclines, and aminoglycosides or a combination thereof. In another embodiment, the additional active agent or treatment is NAC and is administered either intravenously or orally.

### BRIEF DESCRIPTION OF THE DRAWINGS

Figure 1 is the chemical formula of cenicriviroc mesylate.
Figure 2 is a graph comparing the absolute bioavailability, in beagle dogs, of cenicriviroc mesylate compounded as an oral solution with that of cenicriviroc mesylate prepared by wet granulation and mixed with various acid solubilizer excipients.
Figure 3 is a graph of the total impurity and degradant content of different cenicriviroc formulations subjected to accelerated stability testing at 40° C and 75% relative humidity when packaged with a desiccant.
Figure 4 is a dynamic vapor sorption isotherm for different cenicriviroc formulations.
Figure 5 shows the absorption of cenicriviroc from different formulations at three pre-treatment states in beagle dogs.
Figure 6 shows the beagle dog absolute bioavailability of cenicriviroc and lamivudine in combination tablets.
Figure 7 A, B, and C shows the individual body weight data of thiolycollate induced peritonitis model mice after treatment with CVC or dexamethasone. Panel A shows the body weight, Panel B shows the peritoneal cell counts (cells/µL), and Panel C shows the peripheral blood cell counts.
Figure 8 is a graph showing the effect of CVC on macrophages/monocyte recruitment in the mouse thioglycollate induced peritonitis model; N=6 for all groups, except Group 2 where N=8.
Figure 9 is a graph showing the effect of CVC on total leukocytes recrutiment in the mouse thioglycollate induced peritonitis model; N=6 for all groups, except Group 2 where N=8.
Figure 10 is a graph showing the effect of CVC on total leukocytes and macrophages/monocyte recruitment in the mouse thioglycollate induced peritonitis model; N=6 for all groups, except Group 2 were N=8.
Figure 11 shows the individual values of CVC plasma levels for all dose groups in the mouse thioglycollate induced peritonitis model.

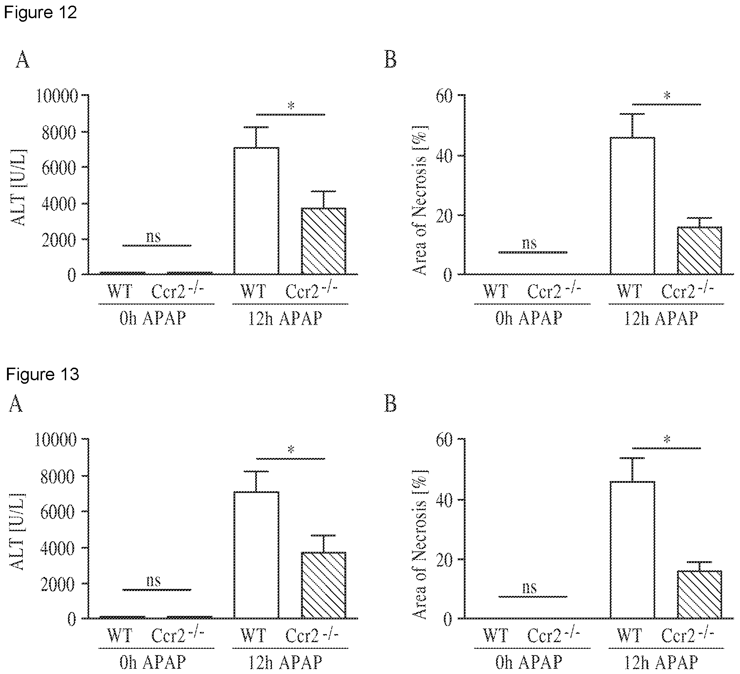

Figure 12 A and B is a graph showing the acetaminophen-induced liver injury as determined by ALT (A)and histology (B) is significantly reduced in *ccr2*^{*-*/*-*} compared to WT mice.
Figure 13 A and B show acetaminophen-induced liver injury as determined by ALT (Panel A) and histology (Panel B) is significantly decreased in CCR2-/- mice compared to wild type mice.

### DETAILED DESCRIPTION

It should be understood that singular forms such as "a," "an," and "the" are used throughout this application for convenience, however, except where context or an explicit statement indicates otherwise, the singular forms are intended to include the plural. All numerical ranges should be understood to include each and every numerical point within the numerical range, and should be interpreted as reciting each and every numerical point individually. The endpoints of all ranges directed to the same component or property are inclusive, and intended to be independently combinable.

### Definitions:

Except for the terms discussed below, all of the terms used in this Application are intended to have the meanings that one of skill in the art at the time of the invention would ascribe to them.

"About" includes all values having substantially the same effect, or providing substantially the same result, as the reference value. Thus, the range encompassed by the term "about" will vary depending on context in which the term is used, for instance the parameter that the reference value is associated with. Thus, depending on context, "about" can mean, for example, ±15%, ±10%, ±5%, ±4%, ±3%, ±2%, ±1%, or ± less than 1%. Importantly, all recitations of a reference value preceded by the term "about" are intended to also be a recitation of the reference value alone. Notwithstanding the preceding, in this application the term "about" has a special meaning with regard to pharmacokinetic parameters, such as area under the curve (including AUC, AUCₜ, and AUC_{∞}) Cₘₐₓ, Tₘₐₓ, and the like. When used in relationship to a value for a pharmacokinetic parameter, the term "about" means from 80% to 125% of the reference parameter.

"Cenicriviroc" refers to the chemical compound (*S*)-8-[4-(2-Butoxyethoxy)phenyl]-1-isobutyl-*N*-(4-{[(1-propyl-1*H*-imidazol-5-yl)methyl]sulfinyl}phenyl)-1,2,3,4-tetrahydro-1-benzazocine-5-carboxamide (structure shown below). Details of the composition of matter of cenicriviroc are disclosed in US Patent Application Publication No. 2012/0232028. Details of related formulations are disclosed in US Application No. 61/823,766.

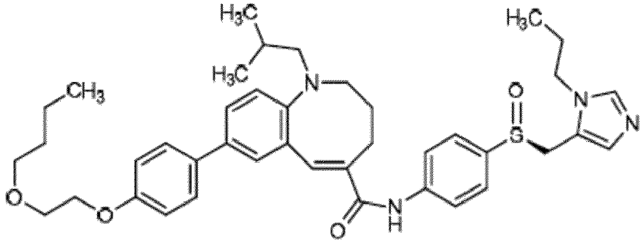

"Compound for use in the present invention" or "the present compound" refers to cenicriviroc or a salt or solvate thereof.

"Substantially similar" means a composition or formulation that resembles the reference composition or formulation to a great degree in both the identities and amounts of the composition or formulation.

"Pharmaceutically acceptable" refers to a material or method that can be used in medicine or pharmacy, including for veterinary purposes, for example, in administration to a subject.

"Salt" and "pharmaceutically acceptable salt" includes both acid and base addition salts. "Acid addition salt" refers to those salts that retain the biological effectiveness and properties of the free bases, which are not biologically or otherwise undesirable, and which are formed with inorganic acids and organic acids. "Base addition salt" refers to those salts that retain the biological effectiveness and properties of the free acids, which are not biologically or otherwise undesirable, and which are prepared from addition of an inorganic base or an organic base to the free acid. Examples of pharmaceutically acceptable salts include, but are not limited to, mineral or organic acid addition salts of basic residues such as amines; alkali or organic addition salts of acidic residues; or a combination comprising one or more of the foregoing salts. The pharmaceutically acceptable salts include salts and the quaternary ammonium salts of the active agent. For example, acid salts include those derived from inorganic acids such as hydrochloric, hydrobromic, sulfuric, sulfamic, phosphoric, and nitric; other acceptable inorganic salts include metal salts such as sodium salt, potassium salt, and cesium salt; and alkaline earth metal salts, such as calcium salt, and magnesium salt, or a combination comprising one or more of the foregoing salts. Pharmaceutically acceptable organic salts includes salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, and HOOC-(CH₂)ₙ-COOH where n is 0-4; organic amine salts such as triethylamine salt, pyridine salt, picoline salt, ethanolamine salt, triethanolamine salt, dicyclohexylamine salt, and N,N'-dibenzyl ethyl enediamine salt; and amino acid salts such as arginate, asparginate, and glutamate; or a combination comprising one or more of the foregoing salts.

In one embodiment, the acid addition salt of cenicriviroc is cenicriviroc mesylate, e.g., (S)-8-[4-(2-Butoxyethoxy)phenyl]-1-isobutyl-*N-*(4-{[(1-propyl-1*H*-imidazol-5-yl)methyl]sulfinyl}phenyl)-1,2,3,4-tetrahydro-1-benzazocine-5-carboxamide monomethanesulfonoate. In one embodiment, the cenicriviroc mesylate is a crystalline material, such as a pale greenish-yellow crystalline powder. In one embodiment, the cenicriviroc mesylate is freely soluble in glacial acetic acid, methanol, benzyl alcohol, dimethylsulfoxide, and N,N-dimethylformamide; soluble in pyridine and acetic anhydride; and sparingly soluble in 99.5% ethanol; slightly soluble in acetonitrile, 1-octanol, and tetrahydrofuran; and practically insoluble in ethyl acetate and diethylether. In one embodiment, the cenicriviroc mesylate is freely soluble in aqueous solution from pH 1 to 2; sparingly soluble at pH 3 and practically insoluble from pH 4 to 13 and in water.

"Solvate" means a complex formed by solvation (the combination of solvent molecules with molecules or ions of the active agent of the present invention), or an aggregate that consists of a solute ion or molecule (the active agent of the present invention) with one or more solvent molecules. In the present invention, the preferred solvate is hydrate.

"Pharmaceutical composition" refers to a formulation of a compound of the disclosure and a medium generally accepted in the art for the delivery of the biologically active compound to mammals, *e.g.,* humans. Such a medium includes all pharmaceutically acceptable carriers, diluents or excipients therefor.

"Treating" includes ameliorating, mitigating, and reducing the instances of a disease or condition, or the symptoms of a disease or condition.

"Administering" includes any mode of administration, such as oral, subcutaneous, sublingual, transmucosal, parenteral, intravenous, intra-arterial, buccal, sublingual, topical, vaginal, rectal, ophthalmic, otic, nasal, inhaled, and transdermal. "Administering" can also include prescribing or filling a prescription for a dosage form comprising a particular compound. "Administering" can also include providing directions to carry out a method involving a particular compound or a dosage form comprising the compound.

"Therapeutically effective amount" means the amount of an active substance that, when administered to a subject for treating a disease, disorder, or other undesirable medical condition, is sufficient to have a beneficial effect with respect to that disease, disorder, or condition. The therapeutically effective amount will vary depending on the chemical identity and formulation form of the active substance, the disease or condition and its severity, and the age, weight, and other relevant characteristics of the patient to be treated. Determining the therapeutically effective amount of a given active substance is within the ordinary skill of the art and typically requires no more than routine experimentation.

Table 1 lists chemokines expressed by liver cells [30].

**Table 1**

| **Cell type** | **Chemokine** |
|---|---|
| Hepatocytes | MCP-1 (CCL2) _{[38]}, MIP-1α (CCL3) _{[74]}, RANTES (CCL5) _{[16,74]}, MIP-3β (CCL19) _{[75]}, SLC (CCL21) _{[75]}, Mig (CXCL9) _{[64]}, IP-10 (CXCL10) _{[64]}, CXCL16 _{[76]}, LEC (CCL16) _{[77]}, IL-8 (CXCL8) _{[78]} and Eotaxin (CCL11) _{[41]} |
| Stellate cells | MCP-1 (CCL2) _{[52,60]}, ,IP-1α (CCL3) _{[60]}, MIP-1β (CCL4) _{[60]}, CX₃CL1 _{[59]}, KC (CXCL1) _{[60]}, MIP-2 (CXCL2) _{[60]}, IP-10 (CXCL10) _{[60]} and SLC (CCL21) _{[70]} |
| Kupffer cells | MCP1 (CCL2) _{[52,38,60,79]}, MIP-1α (CCL3) _{[80]} and MIP-3α (CCL20) _{[56]} |
| Liver endothelial cells | MCP-1 (CCL2) _{[52]}, IL-8 (CXCL8) _{[81,76]}, CXCL16 _{[75]}, Mig (CXCL9) _{[69]}, IP-10 (CXCL10) _{[69]}, CXCL16 _{[65]}, CX₃CL1 _{[82]}, SLC (CCL21) _{[83]}, Eotaxin (CCL11) _{[41]} and TECK (CCL25) _{[73]} |
| *Summarizes selected experimental data from humans and mice/rats regarding the expression of chemokines by different resident hepatic cell populations upon activation or following liver injury. | |
| IP: Interferon-inducible protein; KC: Kupffer cell; LEC: Liver-expressed chemokine; MCP: Monocyte chemoattractant protein; MIP: Macrophage inflammatory protein; SLC: Secondary lymphoid-organ chemokine; TECK: Thymus-expressed chemokine | |

### Peritonitis

Peritonitis (inflammation of the peritoneum) may be localized or generalized, and may result from infection (often due to rupture of a hollow abdominal organ as may occur in abdominal trauma or inflamed appendix) or from a non-infectious process. The symptoms of peritonitis are caused by recruitment of inflammatory factors to the site.

Types of infected peritonitis include, but are not limited to, peritonitis caused by perforation of part of the gastrointestinal tract (e.g. Boerhaave syndrome, peptic ulcer, gastric carcinoma, peptic ulcer, appendicitis, diverticulitis, Meckel diverticulum, inflammatory bowel disease (IBD), intestinal infarction, intestinal strangulation, colorectal carcinoma, meconium peritonitis), or of the gallbladder (cholecystitis, abdominal trauma, ingestion of a sharp foreign body, perforation by an endoscope or catheter, and anastomotic leakage); disruption of the peritoneum, even in the absence of perforation of a hollow viscus (e.g. trauma, surgical wound, continuous ambulatory peritoneal dialysis, and intra-peritoneal chemotherapy.); spontaneous bacterial peritonitis (SBP); or systemic infections (e.g. tuberculosis).

Types of non-infected peritonitis, include but are not limited to, peritonitis caused for example, by leakage of sterile body fluids into the peritoneum, such as blood (e.g., endometriosis, blunt abdominal trauma), gastric juice (e.g., peptic ulcer, gastric carcinoma), bile (e.g., liver biopsy), urine (pelvic trauma), menstruum (e.g., salpingitis), pancreatic juice (pancreatitis), or the contents of a ruptured dermoid cyst; sterile abdominal surgery (e.g. surgery causing localised or minimal generalized peritonitis, which may leave behind a foreign body reaction and/or fibrotic adhesions or a sterile foreign body inadvertently left in the abdomen after surgery); familial Mediterranean fever, TNF receptor associated periodic syndrome, porphyria, and systemic lupus erythematosus.

### Acute liver injury

Many compounds can damage the liver. Well known liver-damaging agents include, but are not limited to, alcohol, Nonsteroidal anti-inflammatory drugs (NSAIDs), Glucocorticoids, Antibiotics (e.g. Isoniazid, Nitrofurantoin, Amoxicillin/clavulanic acid), hydrazine derivative drugs, Industrial toxins such as arsenic, carbon tetrachloride, and vinyl chloride, herbal medicines such as Ackee fruit, Bajiaolian, Camphor, Copaltra, Cycasin, Garcinia, Kava leaves, pyrrolizidine alkaloids, Horse chestnut leaves, Valerian, Comfrey, vitamin A, germander, chaparral leaf, Amanita phylloides, Chinese herbal remedies (e.g. Jin Bu Huan, Ma-huang, Shou Wu Pian, Bai Xian Pi), acetaminophen, Statins, Nicotinic acid (Niacin™), Amiodarone (Cordarone™), Methotrexate (Rheumatrex™, Trexall™), Tacrine (Cognex™), and Disulfiram (Antabuse™).

Liver injury due to excess alcohol consumption is the leading cause of liver disease. It is estimated that consumption of 60-80g per day (about 75-100 ml/day) for 20 years or more in men, or 20g/day (about 25 ml/day) for women significantly increases the risk of hepatitis and fibrosis by 7 to 47%.

Liver injury following acetaminophen intoxication is one of the leading causes of acute liver failure (ALF). Severe acetaminophen hepatotoxicity frequently leads to acute liver failure (ALF). Damage to the liver, or hepatotoxicity, results not from acetaminophen itself, but from one of its metabolites, *N*-acetyl-*p*-benzoquinoneimine (NAPQI)(also known as N-acetylimidoquinone) which depletes the liver's natural antioxidant glutathione and directly damages cells in the liver, leading to liver failure. Liver injury following acetaminophen intoxication causes necrosis of hepatocytes followed by an activation of resident immune cells (e.g. Kupffer cells (KC), hepatocytes, sinusoidal endothelial cells and hepatic stellate cells), release of various chemokines (e.g. CCL2) and immune cell infiltration (e.g. monocytes, macrophages, natural killer (NK), NKT cells and T cells).

Chemokines involved in acute liver injury include, but are not limited to, CCL2, CXCL9, CXCL10, CXCL11, CXCL1, CXCL2, CXCL8, and CXCL12 [1]. For example, and without being bound by theory, when injured, liver cells such as hepatocytes and Kupffer cells secrete CCL2 which leads to monocyte and macrophage infiltration into the liver. Additionally, CCL2 promotes monocytic hematopoiesis in the bone marrow increasing the pool of circulating monocytes/macrophages. The macrophages in the liver then secrete pro-inflammatory cytokines such as TNF-α and IFN-γ [1].

### Embodiments of Therapeutic Utilities:

In one embodiment, the invention relates to a pharmaceutical composition for use in a method which comprises administering cenicriviroc to treat peritonitis. In a further embodiment, the peritonitis is infected peritonitis. In another further embodiment, the infected peritonitis is caused by caused by perforation of part of the gastrointestinal tract (e.g. Boerhaave syndrome, peptic ulcer, gastric carcinoma, peptic ulcer, appendicitis, diverticulitis, Meckel diverticulum, inflammatory bowel disease (IBD), intestinal infarction, intestinal strangulation, colorectal carcinoma, meconium peritonitis), or of the gallbladder (cholecystitis, abdominal trauma, ingestion of a sharp foreign body, perforation by an endoscope or catheter, and anastomotic leakage); disruption of the peritoneum, even in the absence of perforation of a hollow viscus (e.g. trauma, surgical wound, continuous ambulatory peritoneal dialysis, and intra-peritoneal chemotherapy); spontaneous bacterial peritonitis (SBP); or systemic infections (e.g. tuberculosis). In a further embodiment, the peritonitis is non-infected peritonitis. In another further embodiment, the non-infected peritonitis is peritonitis caused for example, by leakage of sterile body fluids into the peritoneum, such as blood (e.g., endometriosis, blunt abdominal trauma), gastric juice (e.g., peptic ulcer, gastric carcinoma), bile (e.g., liver biopsy), urine (pelvic trauma), menstruum (e.g., salpingitis), pancreatic juice(pancreatitis), or even the contents of a ruptured dermoid cyst; sterile abdominal surgery (e.g. surgery causing localized or minimal generalized peritonitis, which may leave behind a foreign body reaction and/or fibrotic adhesions or a sterile foreign body inadvertently left in the abdomen after surgery; familial Mediterranean fever, TNF receptor associated periodic syndrome, porphyria, and systemic lupus erythematosus. In another further embodiment, the CVC is administered in conjunction with one or more peritonitis treatments or agents, including antibiotics, intravenous rehydration and correction of electrolye balance, and/or surgery.

In one embodiment, the invention relates to a pharmaceutical composition for use in a method which comprises administering cenicriviroc to treat acute liver injury induced by acetaminophen, and/or toxins. In a further embodiment, the liver injury involves CCR2 and/or CCL2 expression. In another further embodiment, the CCR2 and/or CCL2 expression is inhibited. In yet another further embodiment, the CCR2 and/or CCL2 expression is inhibited by CVC. In another further embodiment, the CVC is administered in conjunction with one or more treatments or agents for acute liver injury, including either intravenous or oral administration of n-acetylcysteine (Acetylcysteine; NAC), glucocorticoids, corticosteroids, Pentoxifylline, phosphodiesterase inhibitors, anti-TNFa, anti-oxidants, or surgery including liver transplant.

### Dosages and Administration:

A dosage of a particular subject can be determined according to the subject's age, weight, general health conditions, sex, meal, administration time, administration route, excretion rate and the degree of particular disease conditions to be treated by taking into consideration of these and other factors.

The present invention provides a composition for use in a method of treatment as defined in the claims, wherein the cenicriviroc or a salt or solvate thereof is formulated as an oral composition.

The present invention provides a composition for use in a method of treatment as defined in the claims, wherein the cenicriviroc or a salt or solvate thereof is administered once per day or twice per day. The dosage form can be administered for a duration of time sufficient to treat the fibrotic disease or condition.

In the case of oral administration, a daily dosage is in a range of about 5 to 1000 mg, preferably about 10 to 600 mg, and more preferably about 10 to 300 mg, most preferably about 15 to 200 mg as the active ingredient (i.e. as the compound of the invention) per an adult of body weight of 50 kg, and the medicine may be administered, for example, once, or in 2 to 3 divided doses a day.

The cenicriviroc or a salt or solvate thereof may be formulated into any dosage form suitable for oral or injectable administration. When the compound is administered orally, it can be formulated into solid dosage forms for oral administration, for example, tablets, capsules, pills, and granules. It also can be formulated into liquid dosage forms for oral administration, such as oral solutions, oral suspensions, and syrups. The term "tablets" as used herein, refers to those solid preparations which are prepared by homogeneously mixing and pressing the compounds and suitable auxiliary materials into circular or irregular troches, mainly in common tablets for oral administration, including also buccal tablets, sublingual tablets, buccal wafer, chewable tablets, dispersible tablets, soluble tablets, effervescent tablets, sustained-release tablets, controlled-release tablets, and enteric-coated tablets. The term "capsules" as used herein, refers to those solid preparations which are prepared by filling the compounds, or the compounds together with suitable auxiliary materials into hollow capsules or sealing into soft capsule materials. According to the solubility and release property, capsules can be divided into hard capsules (regular capsules), soft capsules (soft shell capsules), sustained-release capsules, controlled-release capsules, and enteric-coated capsules. The term "pills" as used herein, refers to spherical or near-spherical solid preparations which are prepared by mixing the compounds and suitable auxiliary materials via suitable methods, including dropping pills, dragee, and pilule. The term "granules" as used herein, refers to dry granular preparations which are prepared by mixing the compounds and suitable auxiliary materials and have a certain particle size. Granules can be divided into soluble granules (generally referred to as granules), suspension granules, effervescent granules, enteric-coated granules, sustained-release granules, and controlled-release granules. The term "oral solutions" as used herein, refers to a settled liquid preparation which is prepared by dissolving the compounds in suitable solvents for oral administration. The term "oral suspensions" as used herein, refers to suspensions for oral administration, which are prepared by dispersing the insoluble compounds in liquid vehicles, also including dry suspension or concentrated suspension. The term "syrups" as used herein, refers to a concentrated sucrose aqueous solution containing the compounds. The injectable dosage form can be produced by the conventional methods in the art of formulations, and aqueous solvents or non-aqueous solvents may be selected. The most commonly used aqueous solvent is water for injection, as well as 0.9% sodium chloride solution or other suitable aqueous solutions. The commonly used non-aqueous solvent is vegetable oil, mainly soy bean oil for injection, and others aqueous solutions of alcohol, propylene glycol, and polyethylene glycol.

In one embodiment, a pharmaceutical composition comprising cenicriviroc or a salt thereof and fumaric acid is provided. In certain embodiments, the cenicriviroc or salt thereof is cenicriviroc mesylate.

In further embodiments, the weight ratio of cenicriviroc or salt thereof to fumaric acid is from about 7:10 to about 10:7, such as from about 8:10 to about 10:8, from about 9:10 to about 10:9, or from about 95:100 to about 100:95. In other further embodiments, the fumaric acid is present in an amount of from about 15% to about 40%, such as from about 20% to about 30%, or about 25%, by weight of the composition. In other further embodiments, the cenicriviroc or salt thereof is present in an amount of from about 15% to about 40%, such as from about 20% to about 30%, or about 25%, by weight of the composition.

In other further embodiments, the composition of cenicriviroc or a salt thereof and fumaric acid further comprises one or more fillers. In more specific embodiments, the one or more fillers are selected from microcrystalline cellulose, calcium phosphate dibasic, cellulose, lactose, sucrose, mannitol, sorbitol, starch, and calcium carbonate. For example, in certain embodiments, the one or more fillers are microcrystalline cellulose. In particular embodiments, the weight ratio of the one or more fillers to the cenicriviroc or salt thereof is from about 25:10 to about 10:8, such as from about 20:10 to about 10:10, or about 15:10. In other particular embodiments, the one or more fillers are present in an amount of from about 25% to about 55%, such as from about 30% to about 50% or about 40%, by weight of the composition. In other further embodiments, the composition further comprises one or more disintegrants. In more specific embodiments, the one or more disintegrants are selected from cross-linked polyvinylpyrrolidone, cross-linked sodium carboxymethyl cellulose, and sodium starch glycolate. For example, in certain embodiments, the one or more disintegrants is cross-linked sodium carboxymethyl cellulose. In particular embodiments, the weight ratio of the one or more disintegrants to the cenicriviroc or salt thereof is from about 10:10 to about 30:100, such as about 25:100. In other particular embodiments, the one or more disintegrants are present in an amount of from about 2% to about 10%, such as from about 4% to about 8%, or about 6%, by weight of the composition. In other further embodiments, the composition further comprises one or more lubricants. In more specific embodiments, the one or more lubricants are selected from talc, silica, stearin, magnesium stearate, and stearic acid. For example, in certain embodiments, the one or more lubricants are magnesium stearate. In particular embodiments, the one or more lubricants are present in an amount of from about 0.25% to about 5%, such as from about 0.75% to about 3%, or about 1.25%, by weight of the composition.

In other further embodiments, the composition of cenicriviroc or a salt thereof and fumaric acid is substantially similar to that of Table 2. In other further embodiments, the composition of cenicriviroc or a salt thereof and fumaric acid is substantially similar to that of Tables 3 and 4. In other further embodiments, any of the compositions of cenicriviroc or a salt thereof and fumaric acid is produced by a process involving dry granulation. In other further embodiments, any of the compositions of cenicriviroc or a salt thereof and fumaric acid has a water content of no more than about 4% by weight, such as no more than 2% by weight, after six weeks exposure to about 40° C at about 75% relative humidity when packaged with desiccant. In other further embodiments, any of the above-mentioned compositions has a total impurity level of no more than about 2.5%, such as no more than 1.5%, after 12 weeks of exposure to 40° C at 75% relative humidity when packaged with desiccant. In other further embodiments, the cenicriviroc or salt thereof of any of the above-mentioned compositions has a mean absolute bioavailability after oral administration that is substantially similar to the bioavailability of the cenicriviroc or salt thereof in a solution after oral administration. In yet further embodiments, the cenicriviroc or salt thereof has an absolute bioavailability of about 10% to about 50%, such as about 27%, in beagle dogs.

A pharmaceutical formulation is provided that comprises a composition of cenicriviroc or a salt thereof and fumaric acid. In further embodiments, the composition in the formulation can be in the form of a granulate. In other further embodiments, the composition in the formulation is disposed in a capsule shell. In other further embodiments, the composition of the formulation is disposed in a sachet. In other further embodiments, the composition of the formulation is a tablet or a component of a tablet. In still other further embodiments, the composition of the formulation is one or more layers of a multi-layered tablet. In other further embodiments, the formulation comprises one or more additional pharmaceutically inactive ingredients. In other further embodiments, the formulation is substantially similar to that of Table 9. In other further embodiments, a tablet having a composition substantially similar to of Table 9 is provided. In other further embodiments, any of the above embodiments are coated substrates. In another embodiment, methods for preparing any of the above-mentioned embodiments are provided. In further embodiments, the method comprises admixing cenicriviroc or a salt thereof and fumaric acid to form an admixture, and dry granulating the admixture. In other further embodiments, the method further comprises admixing one or more fillers with the cenicriviroc or salt thereof and fumaric acid to form the admixture. In other further embodiments, the method further comprises admixing one or more disintegrants with the cenicriviroc or salt thereof and fumaric acid to form the admixture. In other further embodiments, the method further comprises admixing one or more lubricants with the cenicriviroc or salt thereof and fumaric acid to form the admixture. In other further embodiments, the method further comprises compressing the dry granulated admixture into a tablet. In other further embodiments, the method comprises filling a capsule with the dry granulated admixture.

Further, the compound of the invention can be included or used in combination with blood for transfusion or blood derivatives. In one embodiment, the compound of the disclosure can be included or used in combination with one or more agents that purge latent HIV reservoirs and added to blood for transfusion or blood derivatives. Usually, blood for transfusion or blood derivatives are produced by mixing blood obtained form plural persons and, in some cases, uninfected cells are contaminated with cells infected with HIV virus. In such a case, uninfected cells are likely to be infected with HIV virus. When the compound of the present disclosure is added to blood for transfusion or blood derivatives along with one or more agents that purge latent HIV reservoirs, infection and proliferation of the virus can be prevented or controlled. Especially, when blood derivatives are stored, infection and proliferation of the virus is effectively prevented or controlled by addition of the compound of the present disclosure. In addition, when blood for transfusion or blood derivatives contaminated with HIV virus are administered to a person, infection and proliferation of the virus in the person's body can be prevented by adding the compound of the disclosure to the blood or blood derivatives in combination with one or more agents that purge latent HIV reservoirs. For example, usually, for preventing HIV infectious disease upon using blood or blood derivatives by oral administration, a dosage is in a range of about 0.02 to 50 mg/kg, preferably about 0.05 to 30 mg/kg, and more preferably about 0.1 to 10 mg/kg as the CCR5/CCR2 antagonist per an adult of body weight of about 60 kg, and the medicine may be administered once or 2 to 3 doses a day. As a matter of course, although the dosage range can be controlled on the basis of unit dosages necessary for dividing the daily dosage, as described above, a dosage of a particular subject can be determined according to the subject's age, weight, general health conditions, sex, meal, administration time, administration route, excretion rate and the degree of particular disease conditions to be treated by taking into consideration of these and other factors. In this case, the administration route is also appropriately selected and, the medicine for preventing HIV infectious disease of the present disclosure may be added directly to blood for transfusion or blood derivatives before transfusion or using blood derivatives. In such a case, desirably, the medicine of the present disclosure is mixed with blood or blood derivatives immediately to 24 hours before, preferably immediately to 12 hours before, more preferably immediately to 6 hours before transfusion or using blood derivatives.

Aside from blood for transfusion or blood derivatives, when the compositions of the disclosure is administered together with the blood for transfusion or blood derivatives and/or other active agents, the medicine is administered preferably at the same time of, to 1 hour before transfusion or using the blood derivatives. More preferably, for example, the medicine is administered once to 3 times per day and the administration is continued 4 weeks.

### Combination Therapy:

The compound of the disclosure may be used alone or in combination with one or more additional active agents. The one or more additional active agents may be any compound, molecule, or substance which can exert therapeutic effect to a subject in need thereof. The one or more additional active agents may be "co-administered", i.e., administered together in a coordinated fashion to a subject, either as separate pharmaceutical compositions or admixed in a single pharmaceutical composition. By "co-administered", the one or more additional active agents may also be administered simultaneously with the present compound, or be administered separately with the present compound, including at different times and with different frequencies. The one or more additional active agents may be administered by any known route, such as orally, intravenously, intramuscularly, nasally, subcutaneously, intra-vaginally, and intra-rectally; and the therapeutic agent may also be administered by any conventional route. In many embodiments, at least one and optionally both of the one or more additional active agents may be administered orally.

These one or more additional active agents include, but are not limited to, one or more anti-fibrotic agents, antiretroviral agents, immune system suppressing agents, CCR2 and/or CCR5 inhibitors or treatments, n-acetylcysteine (Acetylcysteine; NAC), glucocorticoids, corticosteroids, Pentoxifylline, phosphodiesterase inhibitors, anti-TNFa agents, anti-oxidants, and antibiotics. When two or more medicines are used in combination, dosage of each medicine is commonly identical to the dosage of the medicine when used independently, but when a medicine interferes with metabolism of other medicines, the dosage of each medicine is properly adjusted. Each medicine may be administered simultaneously or separately in a time interval for example of less than 12 hours, 24 hours, and 36 hours. A dosage form as described herein, such as a capsule, can be administered at appropriate intervals. For example, once per day, twice per day, and three times per day. In particular, the dosage form is administered for example, once or twice per day. Even more particularly, the dosage form is administered once per day. In one embodiment, the one or more antiretroviral agents include, but are not limited to, entry inhibitors, nucleoside reverse transcriptase inhibitors, nucleotide reverse transcriptase inhibitors, non-nucleoside reverse transcriptase inhibitors, protease inhibitors, integrase inhibitors, maturation inhibitors, and combinations thereof. In one embodiment, the one or more additional antiretroviral agents include, but are not limited to, lamivudine, efavirenz, raltegravir, vivecon, bevirimat, alpha interferon, zidovudine, abacavir, lopinavir, ritonavir, tenofovir, tenofovir disoproxil, tenofovir prodrugs, emtricitabine, elvitegravir, cobicistat, darunavir, atazanavir, rilpivirine, dolutegravir, and a combination thereof.

In one embodiment, the one or more immune system suppressing agents include, but are not limited to, cyclosporine, tacrolimus, prednisolone, hydrocortisone, sirolimus, everolimus, azathioprine, mycophenolic acid, methotrexate, basiliximab, daclizumab, rituximab, anti-thymocyte globulin, anti-lymphocyte globulin, and a combination thereof.

In one embodiment, the one or more antibiotics include, but are not limited to, Penicillins (e.g. penicillin and amoxicillin); Cephalosporins (e.g. cephalexin); Macrolides (e.g. erythromycin, clarithromycin, and azithromycin); Fluoroquinolones (e.g. ofloxacin, levofloxacin , and ofloxacin); Sulfonamides (e.g. co-trimoxazole and trimethoprim); Tetracyclines (e.g. tetracycline and doxycycline); Aminoglycosides (e.g. gentamicin and tobramycin).

In one embodiment, the one or more glucocorticoids include but are not limited to, Triamcinolone, methylprednisolone systemic, betamethasone, budesonide, prednisolone, prednisone, hydrocortisone, dexamethasone, and/or cortisone.

In one embodiment, the one or more anti-TNFa agents include, but are not limited to, Infliximab, Etanercept, Adalimumab, Certolizumab, and/or Golimumab.

In one embodiment, the one or more phosphodiesterase inhibitors include, but are not limited to, sildenafil, tadalafil, and/or vardenafil.

The following Examples further illustrate the present invention in detail but are not to be construed to limit the scope thereof.

### EXAMPLES

### Example 1 - Cenicriviroc mesylate compositions

A series of cenicriviroc mesylate compositions that were identical except for the identity of the acid solubilizer were prepared by wet granulation in a Key 1L bowl granulator, followed by tray drying, sieving, mixing and compression into tablets on a Carver press. The composition of the formulations is shown in Table 2.

**Table 2**

| **Components** | **Unit Formula (mg/unit)** | | | |
|---|---|---|---|---|
| | **Ex. 1a Citric Acid** | **Ex. 1b Fumaric Acid** | **Ex. 1c Maleic Acid** | **Ex. 1d Sodium Bisulfate** |
| Cenicriviroc Mesylate | 28.45 | 28.45 | 28.45 | 28.45 |
| Mannitol | 7.88 | 7.88 | 7.88 | 7.88 |
| Hydroxypropyl Cellulose | 2.62 | 2.62 | 2.62 | 2.62 |
| Croscarmellose Sodium | 1.75 | 1.75 | 1.75 | 1.75 |
| Croscarmellose Sodium | 1.75 | 1.75 | 1.75 | 1.75 |
| Citric Acid | 43.75 | - | - | - |
| Fumaric Acid | - | 43.75 | - | - |
| Maleic Acid | - | - | 43.75 | - |
| Sodium Bisulfate | - | - | - | 43.75 |
| Silicon Dioxide | 0.43 | 0.43 | 0.43 | 0.43 |
| Magnesium Stearate | 0.88 | 0.88 | 0.88 | 0.88 |
| Total | 87.5 | 87.5 | 87.5 | 87.5 |

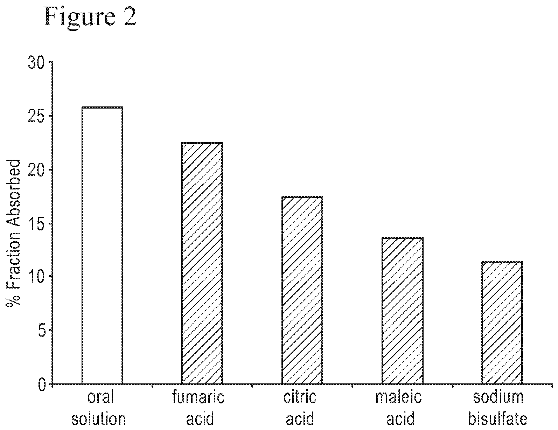

The tablets were administered to beagle dogs. An oral solution was also administered as a control. The absolute bioavailabilities of the formulations and of the oral solution were determined, and are shown in Figure 2. The result shows that the cenicriviroc mesylate with fumaric acid has a significantly higher bioavailability than any of the other solubilizers tested.

### Example 2: Cenicriviroc mesylate compositions

Cenicriviroc mesylate, fumaric acid, microcrystalline cellulose, cross-linked sodium carboxymethyl cellulose, and magnesium stearate were admixed, dry granulated, milled, blended with extragranular microcrystalline cellulose, cross-linked sodium carboxymethyl cellulose, and magnesium stearate and compressed into tablets having a hardness greater than 10 kP and friability less than 0.8% w/w. The resulting tablets had the composition shown in Table 3.

**Table 3**

| Components | **Unit Formula (mg/unit)** | | | | |
|---|---|---|---|---|---|
| | **Ex. 2a** | **Ex. 2b** | **Ex. 2c** | **Ex. 2d** | **Ex. 2e** |
| Cenicriviroc Mesylate | 170.69^{a} | 170.69^{a} | 170.69^{a} | 170.69^{a} | 170.69^{a} |
| Fumaric Acid | 160.00 | 160.00 | 160.00^{b} | 160.00 | 80.00 |
| Microcrystalline Cellulose | 252.68 | 272.18 | 272.18 | 272.18 | 66.35 |
| Crospovidone | - | - | - | 19.50 | - |
| Croscarmellose Sodium | 58.50 | 39.00 | 39.00 | 19.50 | 20.70 |
| Magnesium Stearate | 8.13 | 8.13 | 8.13 | 8.13 | 2.55 |
| Total | 650.0 | 650.0 | 650.0 | 650.0 | 340.0 |

| | | | | | |
|---|---|---|---|---|---|
| a. Equivalent to 150 mg cenicriviroc freebase. b. Added in the extragranular portion of the powder blend. | | | | | |

By way of illustration, the concentration percentage and mass per tablet of the components in Example 2b (i.e., Ex. 2b) are given in Table 4.

**Table 4**

| **Component** | **Concentration (% w/w)** | **Mass (mg) per tablet** |
|---|---|---|
| Cenicriviroc mesylate | 26.26 | 170.69^{a} |
| Fumaric acid | 24.62 | 160.00 |
| Microcrystalline cellulose | 41.87 | 272.18 |
| Cross-linked sodium carboxymethyl cellulose | 6.00 | 39.00 |
| Magnesium stearate | 1.25 | 8.13 |
| Total | 100.0 | 650.0 |

| | | |
|---|---|---|
| ^{a} equivalent to 150 mg cenicriviroc free base | | |

### Example 3: Cenicriviroc mesylate compositions

Cenicriviroc mesylate, microcrystalline cellulose, cross-linked sodium carboxymethyl cellulose, and magnesium stearate were admixed, dry granulated, dried, milled, blended with extragranular microcrystalline cellulose, cross-linked sodium carboxymethyl cellulose, fumaric acid, colloidal silicon dioxide, and magnesium stearate and compressed into tablets having a hardness greater than 10 kP and friability less than 0.8% w/w. The resulting tablets had the composition shown in Table 5.

**Table 5**

| **Component** | **Concentration (% w/w)** | **Mass (mg) per tablet** |
|---|---|---|
| Cenicriviroc mesylate | 26.26 | 28.45^{a} |
| Fumaric acid | 24.62 | 26.67 |
| Microcrystalline cellulose | 41.87 | 45.36 |
| Cross-linked sodium carboxymethyl cellulose | 6.00 | 39.00 |
| Magnesium stearate | 1.25 | 1.35 |
| Total | 100.0 | 108.3 |

| | | |
|---|---|---|
| ^{a} equivalent to 25 mg cenicriviroc free base | | |

Notably, the formulation of Table 5 has the same ratio of components as that of Table 3b, and differs only in the total amount of the components that are used for each tablet. Thus, Table 4 shows tablets with 150 mg cenicriviroc (based on free base), whereas Table CC-1 shows tablets with 25 mg cenicriviroc (based on free base) with the same ratio of components as the 150 mg tablets of Example 2b, shown in Table 4.

### Example 4 - Reference

The citric acid based formulation of Table 6 was prepared as follows. Cenicriviroc, hydroxypropyl cellulose, mannitol, and cross-linked sodium carboxymethyl cellulose were admixed, wet granulated, dried, milled, and blended with microcrystalline cellulose, cross-linked sodium carboxymethyl cellulose, citric acid, colloidal silicon dioxide, talc, and magnesium stearate. The resulting blend was compressed into tablets having a hardness greater than 10 kP and friability less than 0.8% w/w. The tablets were coated with hydroxypropyl methylcellulose, polyethylene glycol 8000, titanium dioxide, and yellow iron oxide. The coated tablets thus produced were substantially identical to those disclosed in U.S. Patent Application Publication No. 2008/031942 (see, e.g., Table 3).

**Table 6**

| **Component** | **mg/tablet** | **%w/w** |
|---|---|---|
| Cenicriviroc mesylate | 28.91 | 4.68 |
| Mannitol | 341.09 | 56.85 |
| Microcrystalline cellulose | 80.00 | 12.94 |
| Colloidal silicon dioxide | 12.00 | 2.00 |
| Citric acid anhydrous | 75.00 | 12.14 |
| Hydroxypropyl cellulose | 12.00 | 1.94 |
| Cross-linked sodium carboxymethyl cellulose | 30.00 | 4.85 |
| Talc | 12.00 | 1.94 |
| Magnesium stearate | 9.00 | 1.46 |
| Hydroxypropyl methylcellulose | 11.71 | 1.89 |
| Polyethylene glycol 8000 | 2.69 | 0.44 |
| Titanium dioxide | 3.03 | 0.49 |
| Yellow iron oxide | 0.57 | 0.09 |

### Example 5- Reference

Cenicriviroc and hypromellose acetate succinate were dissolved in methanol and spray dried into a fine powder containing 25% cenicriviroc by weight (based on the weight of cenicriviroc free base). The powder was admixed with colloidal silicon dioxide, microcrystalline cellulose, mannitol, sodium lauryl sulfate, cross-linked sodium carboxymethyl cellulose, and magnesium stearate. The admixture was compressed into tablets having a hardness greater than 10 kP and friability less than 0.8% w/w. The final composition of the tablets is shown in Table 7.

**Table 7**

| **Component** | **Weight %** | **Mass (mg)** |
|---|---|---|
| Cenicriviroc (as mesylate salt) | 8.33 | 50.00 |
| Hypromellose acetate succinate | 25.00 | 150.00 |
| Sodium lauryl sulfate | 2.00 | 12.00 |
| Cross-linked sodium carboxymethyl cellulose | 6.00 | 36.00 |
| Microcrystalline cellulose | 27.83 | 167.00 |
| Mannitol | 27.83 | 167.00 |
| Colloidal silicon dioxide | 1.00 | 6.00 |
| Magnesium stearate | 2.00 | 12.00 |
| Total | 100.0 | 600.0 |

### Example 6: Bioavailibility of CVC formulation

The absolute bioavailability of the tablets of Example 3 in beagle dogs was compared to that of the tablets of Examples 4 and 5, as well as to both an oral solution of cenicriviroc mesylate and a gelatin capsule containing cenicriviroc mesylate powder. The results are shown in Table 8.

**Table 8**

| **Component** | **Absolute bioavailability(%)** |
|---|---|
| Oral Solution | 25.8 |
| Powder in capsule | 6.4 |
| Example 3 | 26.6 |
| Example 4 | 21.1 |
| Example 5 | 12.4 |

This example demonstrates that the bioavailability of cenicriviroc in dry granulated tablets with fumaric acid (Ex. 3) is substantially similar to that of an oral solution, and is significantly higher than the bioavailability of cenicriviroc in wet granulated tablets with fumaric (Ex. 1b) or citric acid (Ex. 4), and over double that of cenicriviroc in tablets with amorphous cenicriviroc in a spray dried dispersion with HPMC-AS (Ex. 5). These results are surprising, because there was no reason to suspect that dry granulation of crystalline API provides a significant increase in bioavailability over wet granulation and amorphous spray dried dispersions. This is especially so because amorphous spray dried dispersions are frequently used to increase the bioavailability of poorly water soluble drugs. These results are also surprising because fumaric acid has a slower dissolution time than citric acid and was used at a lower mass ratio of acid relative to CVC API (3:1 for citric acid: API versus 1.06:1 fumaric acid : API). Hence it was therefore surprising that fumaric acid proved to be a more effective solubilizer than citric acid for CVC.

### Example 7: Accelerated stability of CVC formulation

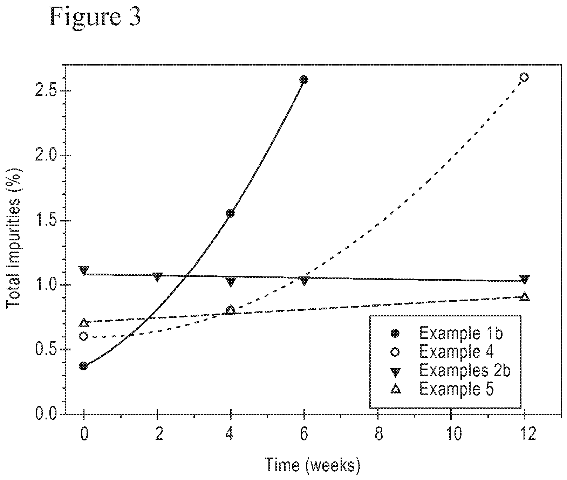

The accelerated stability of the tablets of Example 2b was compared to that of the tablets of Examples 1b, 4, and 5 via exposure to an environment of 75% relative humidity at 40° C. All tablets were packaged with a desiccant during the study. As shown in Figure 3, the tablets of Examples 2b are surprisingly much more stable than the other wet granulated tablets, and similarly stable as the spray dried dispersion tablets. This difference in stability between the tablets of Examples 2b and Example 4 is particularly surprising since the only significant difference between the two is the method of making the formulations (dry granulation vs. wet granulation). These results are also surprising, because it was not previously known that the method of granulation could have an effect on both cenicriviroc bioavailability and stability.

### Example 8: Stability of CVC formulation

The stability of the tablets of Examples 2 and 3 was tested by exposing the tablets to an environment of 75% relative humidity at 40° C for six weeks. All tablets were packaged with a desiccant during the study. The results are shown in Table 9, which shows that the tablets are very stable under these conditions.

**Table 9**

| **Time (Weeks)** | **Water content (%)** | **Strength (%)** | **Total Impurities (%)** |
|---|---|---|---|
| 0 | 1.5 | 99.1 | 1.2 |
| 2 | 1.4 | 99.2 | 1.1 |
| 4 | 1.4 | 98.0 | 1.0 |
| 6 | 1.4 | 98.6 | 1.0 |

### Example 9: Stability of CVC formulations

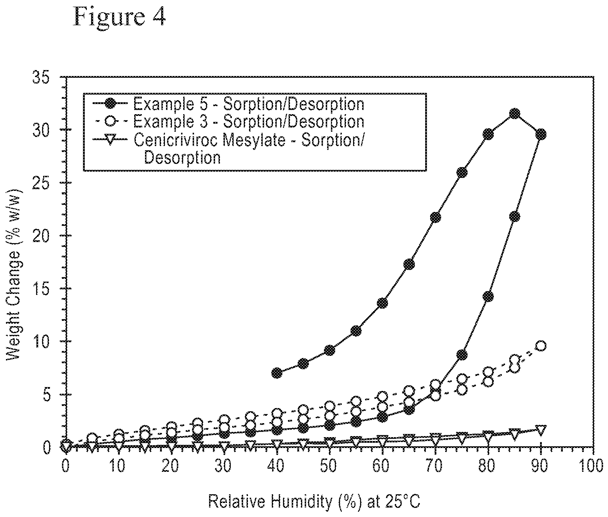

Dynamic vapor sorption isotherms at 25° C correlate to the stability of the tablets of Examples 3 and 4 with that of cenicriviroc mesylate. Sorption was performed from 0% relative humidity to 90% relative humidity at 5% intervals. At each interval, each sample was equilibrated for no less than 10 minutes and no longer than 30 minutes. Equilibration was stopped when the rate of mass increase was no more than 0.03% w/w per minute or after 30 minutes, whichever was shorter. The result, which appears in Figure 4, shows that tablets of Example 2b are significantly more stable than those of Example 4. This result is consistent with Example 3 being significantly less hygroscopic than Example 4. The increased hygroscopicity of Example 4, in comparison to Examples 2b, can be associated with a higher mobile water content which can in turn cause partial gelation and subsequent decreased stability of Example 4.

### Example 10: Bioavailability of CVC formulations

The bioavailability of the tablets of Example 3 was compared to that of Example 5 and cenicriviroc mesylate powder in a gelatin capsule in different stomach states in beagle dogs. The bioavailability was tested under different pre-treatment states, each of which alters the gastric pH. Specifically, pentagastric pretreatment provides the lowest pH, no treatment provides an intermediate pH, and famotidine treatment provides the highest pH.

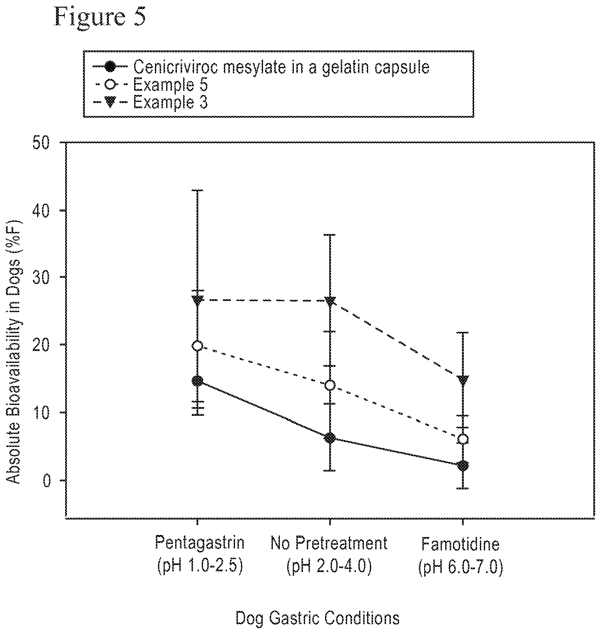

The result, which appears in Figure 5, shows that the tablets of Example 3 have a higher bioavailability under all conditions that were tested. The bioavailability of Example 3 varied less between pentagastrin treated and untreated dogs, whereas Example 4 showed a significant loss of bioavailability in fasted, non-treated dogs (intermediate gastric pH) compared to that in pentagastrin treated dogs (lowest gastric pH). Pretreatment with famotidine, an H2 receptor agonist that suppresses stomach acidity and raises gastric pH decreased bioavailability for all samples, however, the reduction for Example 3 was much less than that for Example 4.

These results demonstrate an additional unexpected benefit of dry granulated cenicriviroc compositions with fumaric acid. Specifically, the pharmacokinetics of such formulations do not vary as much as those of the spray dried dispersion (Example 4) when administered across a the full range of potential human gastric pH conditions. This result is unexpected and surprising, because the bioavailability of other weakly basic antiretroviral drugs, such as atazanavir, is greatly affected by the gastric pH. For such drugs, changes in gastric pH, which can be caused by a disease or medical condition, such as achlorohydric patients, or by co-administration of drugs such as antacids, proton pump inhibitors, or H2 receptor agonists, can lower the bioavailability to sub-therapeutic levels. These results showing that the dry granulated, fumaric acid based cenicriviroc mesylate formulation of Example 3 is less prone to bioavailability changes as the gastric pH changes shows that Example 3 is a more robust formulation that can be used in patients who have or are likely to have varying gastric pH levels.

### Examples 11a-11c: Preparation of Cenicriviroc mesylate and lamivudine formulations

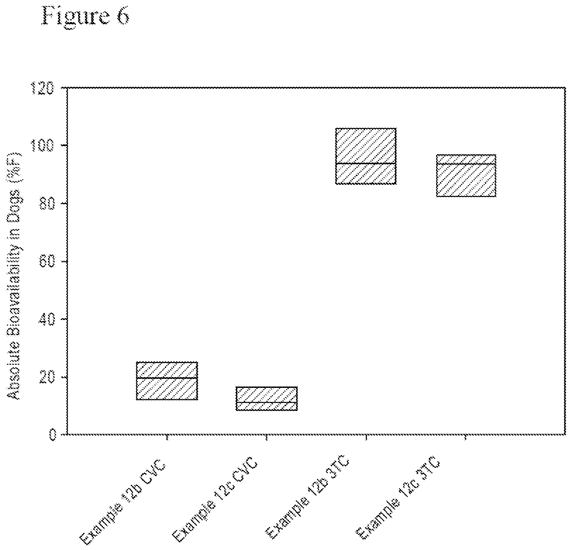

The formulations of cenicriviroc mesylate and lamivudine of Table 10 were prepared as follows. First, the intragranular components were admixed and dry granulated to form a composition as a dry granulated admixture. This dry granulated admixture was then further admixed with the extragranular components to form a mixture. The mixture was compressed into tablets. The absolute bioavailability of the cenicriviroc (CVC) and lamivudine (3TC) in beagle dogs in the 150 mg CVC strength tablets (Examples 11b and 11c) were measured. The results are shown in Figure 6.

**Table 10**

| | **Example 12a** | | **Example 12b** | | **Example 12c** | |
|---|---|---|---|---|---|---|
| | **25 mg cenicriviroc and 300 mg lamivudine** | | **150 mg cenicriviroc and 300 mg lamivudine** | | **150 mg cenicriviroc and 300 mg lamivudine** | |
| | **% w/w** | **mg/tablet** | **% w/w** | **mg/tablet** | **% w/w** | **mg/tablet** |
| **Intragranular Components** | | | | | | |
| Cenicriviroc mesylate | 5.69 | 28.45 | 17.97 | 170.69 | 21.34 | 170.69 |
| Fumaric Acid | 5.33 | 26.67 | 16.84 | 160.00 | 20.00 | 160.00 |
| Microcrystalline cellulose | 5.82 | 29.11 | 18.39 | 19.50 | 2.64 | 21.10 |
| Cross-linked sodium carboxymethyl cellulose | 0.65 | 3.25 | 2.05 | 19.50 | 2.64 | 21.10 |
| Magnesium stearate | 0.16 | 0.81 | 0.51 | 4.88 | 0.53 | 4.20 |

| **Extragranular Components** | | | | | | |
|---|---|---|---|---|---|---|
| Lamivudine (3TC) | 60.00 | 300.00 | 31.58 | 600.00 | 37.50 | 300.00 |
| Microcrystalline cellulose | 16.34 | 81.71 | 6.39 | 60.75 | 3.78 | 30.21 |
| Cross-linked sodium carboxymethyl cellulose | 5.00 | 25.00 | 5.26 | 50.00 | 5.00 | 40.00 |
| Magnesium stearate | 1.00 | 5.00 | 1.00 | 9.50 | 1.00 | 8.00 |
| **Total per tablet** | 100.00 | 500.00 | 100.00 | 950.00 | 100.00 | 800.00 |

### Example 12 - Effect of Cenicriviroc in the Thioglycollate Induced Peritonitis Model in Mice

Summary: The mouse thioglycollate induced peritonitis model is a commonly used preclinical model to assess the recruitment of anti-inflammatory cells and to assess the activation of macrophages. The objective of this study was to evaluate the effects of cenicriviroc (CVC), in the mouse thioglycollate-induced peritonitis (TIP) model. From Days 1 to 5 animals received the vehicle, CVC, or positive control by oral gavage at a dose volume of 10 mL/kg. Twice daily (BID) dosing in Groups 2 to 5 was separated by approximately 12 hours. On Day 4, 2 hours after dosing of vehicle, CVC, or positive control (2 hours after first dose for BID groups), animals received an intraperitoneal injection of saline (Group 1) or 3.85% thioglycollate (TG) at a volume of 1mL/animal.

**Table 11 - Experimental Design**

| Group | Group treatment | Total Dose (mg/kg/day) | Dose (mg/kg/dose) | Dose volume (mL/kg) | Formulation conc. (mg/mL) | No. of Males |
|---|---|---|---|---|---|---|
| 1 | Non-disease control | 0 | 0 | 10 | 0 | 6 |
| 2 | TG - Control BID^{a} | 0 | 0 (BID) | 10 | 0 | 8 |
| 3 | TG - CVC BID | 5 | 2.5 (BID) | 10 | 0.25 | 6 |
| 4 | TG - CVC BID | 20 | 10 (BID) | 10 | 1 | 6 |
| 5 | TG - CVC BID | 100 | 50 (BID) | 10 | 5 | 6 |
| 6 | TG - CVC QD | 20 | 20 | 10 | 2 | 6 |
| 7 | TG-Dexamethasone - QD | 1 | 1 | 10 | 0.1 | 6 |

| | | | | | | |
|---|---|---|---|---|---|---|
| TG = Thioglycollate ^{a} Control animals received the vehicle for CVC (0,5% (w/v) methylcellulose. 1% Tween® 80 (pH ∼ 1.3) in DI Water) | | | | | | |

The following parameters and end points were evaluated in this study: clinical signs, body weights, peritoneal lavage cell counts, peripheral blood cell counts and pharmacokinetic evaluation. There were no treatment related clinical signs or effects on body weights. There were no clear changes observed in circulating blood cell counts. Following CVC administration in the murine thioglycollate peritonitis model, a clear dose related decrease was observed in peritoneal inflammatory cell recruitment, both on total leukocyte and mononuclear populations. There was a dose related increase in plasma exposures of CVC at the three dose levels. CVC appeared to be more efficacious when given BID compared to QD, in line with the higher plasma concentrations achieved with BID dosing and the known short half-life in mice (∼2 hours).

Study Design: From Days 1 to 5, animals received the vehicle (0.5% (w/v) methylcellulose, 1% Tween® 80 (pH∼ 1.3) in DI Water, CVC, or positive control (dexamethasone (Dex), lot 071M1180V) by oral gavage at a dose volume of 10 mL/kg. BID dosing in Groups 2 to 5 were separated by approximately 12 hours. The formulations were prepared once for the entire study and aliquoted for each day of dosing.

On Day 4, 2 hours after dosing of vehicle, CVC, or positive control (2 hours after first dose for BID groups), animals received an intraperitoneal injection of saline (Group 1) or 3.85% thioglycollate at a volume of 1mL/animal (Groups 2-7). Dexamethasone was used as a positive control in this experiment as it is a corticosteroid known to reduce inflammation in a variety of animal models. Murine inflammation models indicate that the effective dose range for dexamethasone, when administered orally, is 0.3 to 3 mg/kg QD. On this basis, an intermediate dose of 1 mg/kg was chosen for this particular study.

In-life Procedures, Observations, and Measurements: Morbidity/mortality checks were performed at least once daily. Body weights were recorded prior to dosing and prior to necropsy.

Terminal Procedures: All animals were euthanized, 48 hours following thioglycollate injection, by CO2 asphyxiation and a peritoneal lavage was performed with 2.5mL of ice-cold sterile Ca2+/Mg2+-free PBS containing 0.01M EDTA. The lavage was repeated with a second injection of 2.5mL via the initial puncture site and the samples were pooled for each animal.

The lavage fluid was placed on wet ice pending processing for total and differential cell counts using the Advia analyzer. Following the lavage collection, a blood sample (0.7mL) was collected from each mouse by intracardiac puncture or from the vena cava (collection time was recorded). 0.5mL of blood was placed in an EDTA tube, transferred to clinical pathology and analyzed for differential cell counts using the Advia analyzer. 0.2mL of blood was placed in a K2EDTA tube and processed to plasma. The plasma was collected by centrifugation (3000 rpm for 10 minutes at 4°C) and placed on dry ice. The samples were analyzed to determine CVC plasma levels. After blood collection, the animals were then exsanguinated by sectioning of the abdominal aorta.

Statistical Analysis: One-way ANOVA with post-hoc Dunnett's statistical analysis was performed on differential cell counts obtained from lavage and blood samples. The analysis was performed in comparison to Group 2.

### RESULTS

Mortality: No mortality occurred during the conduct of the study.

Clinical Observations: No unusual clinical signs were noted during the conduct of the study.

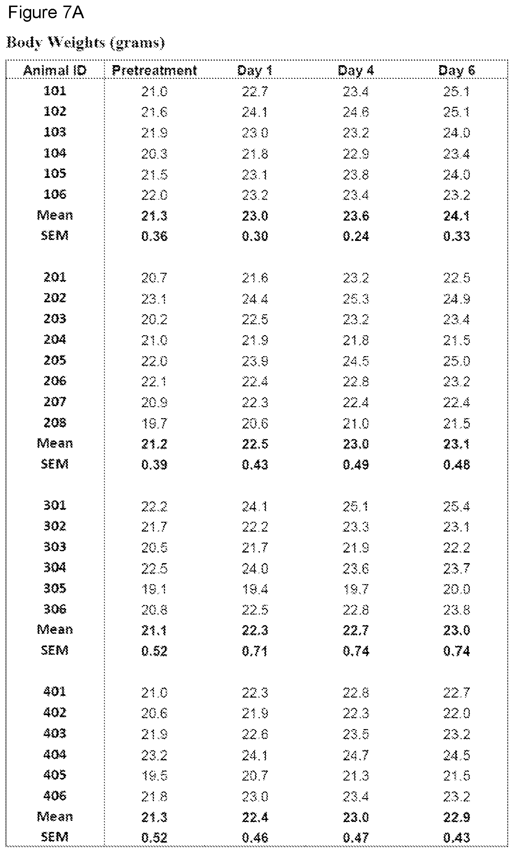

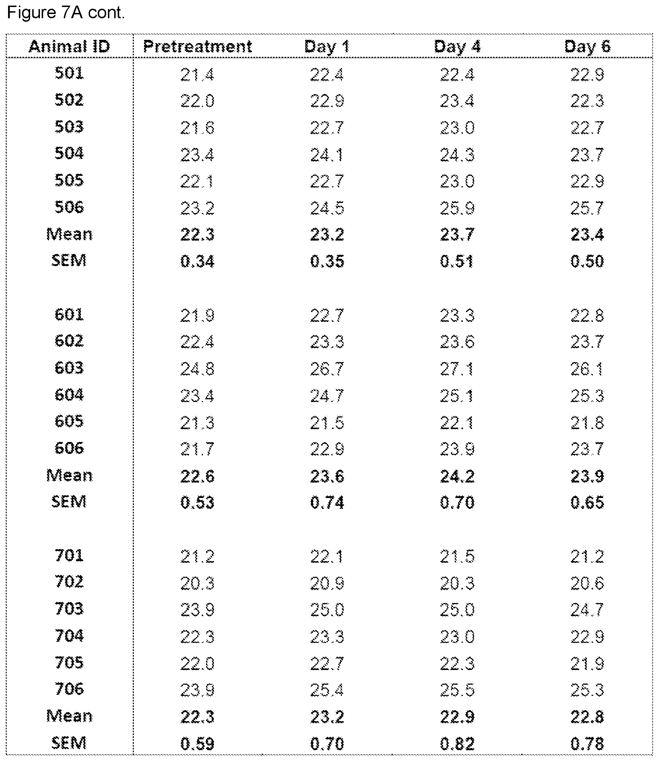

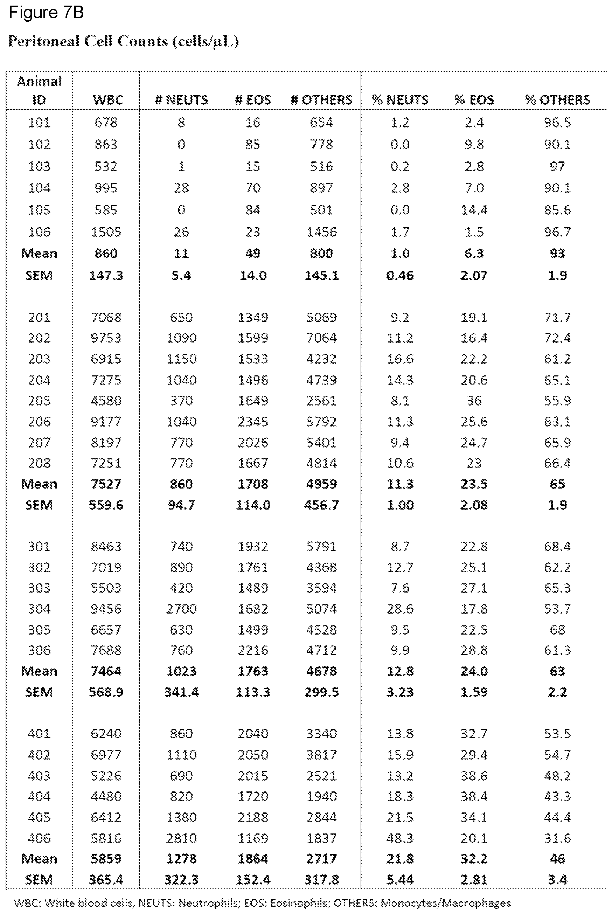

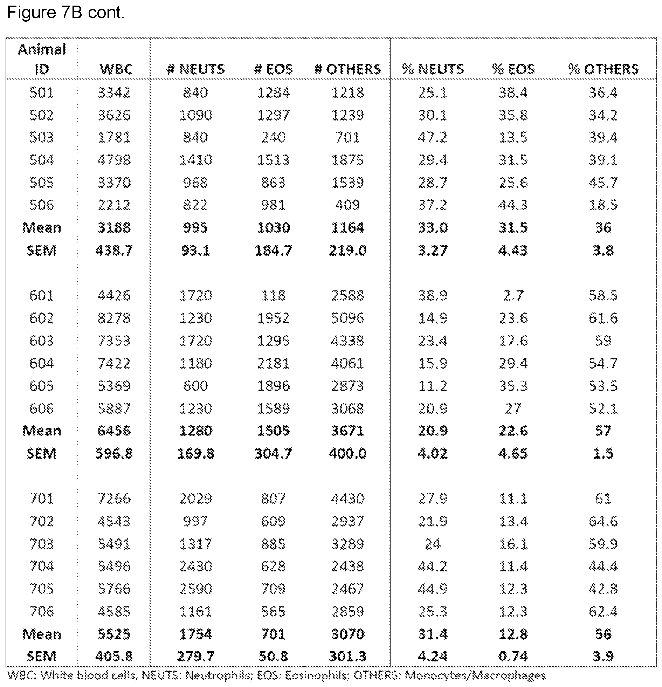

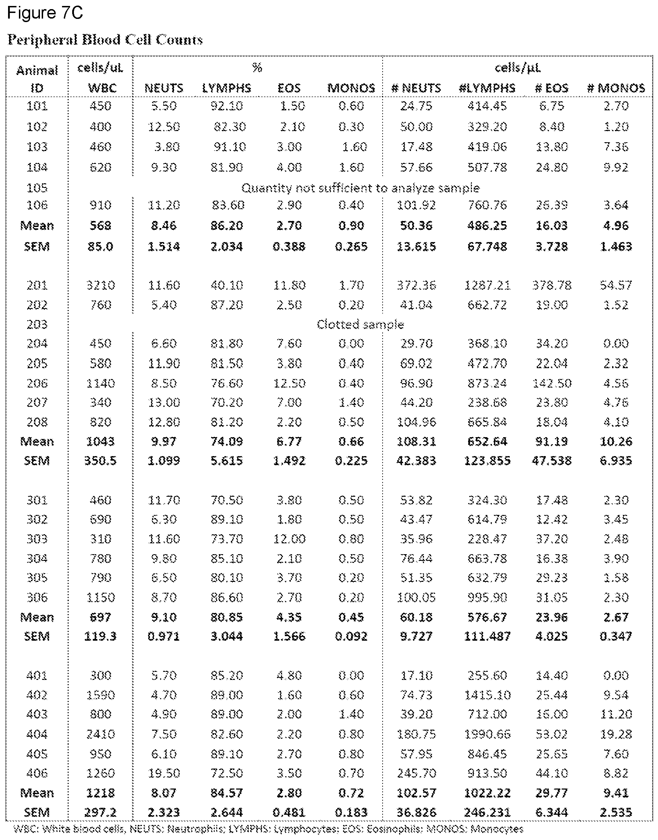

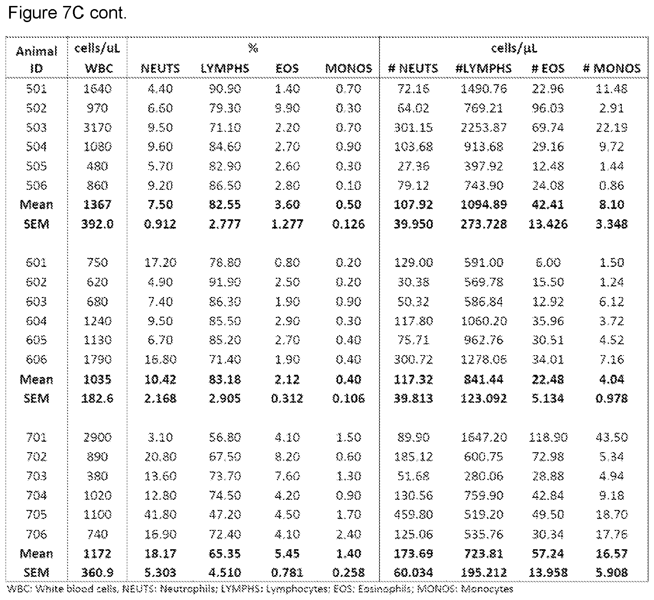

Body Weights: There were no notable changes in body weights following CVC administration (Days 1 to 6). Body weights from the dexamethasone group (Group 7) decreased slightly from Days 1 to 6. The body weight data is presented in Figure 7A, B, and C.

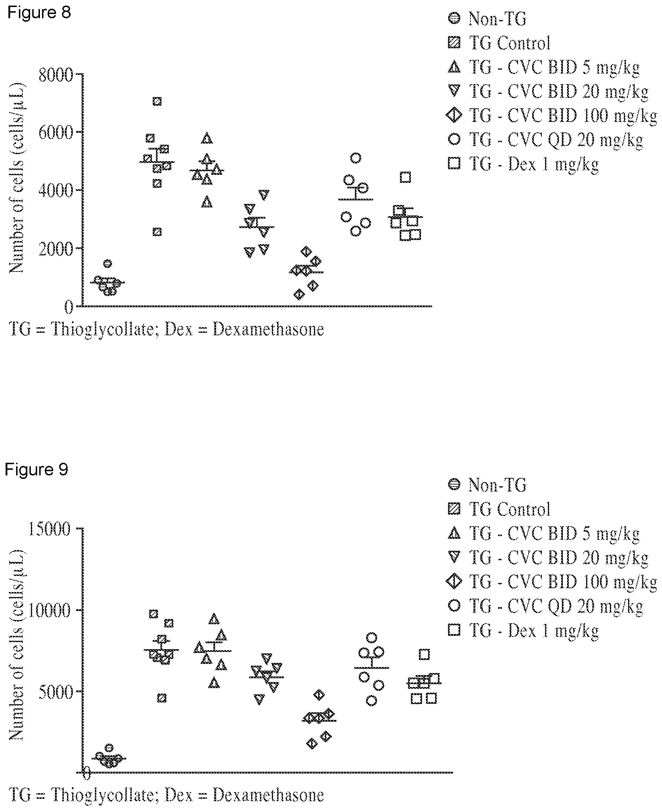

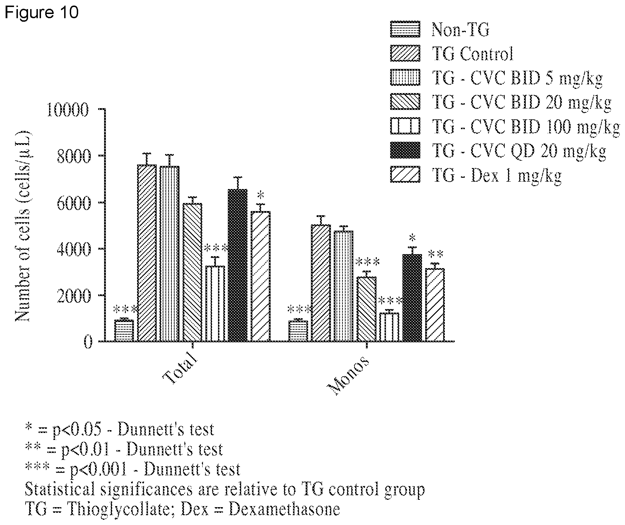

Peritoneal Lavage: Increases in monocytes/macrophages and total leukocytes were observed in all groups induced with thioglycollate. A clear dose-related effect of CVC was observed when compared to TG control (Group 2), attaining statistical significance (p<0.05) at doses ≥ 20 mg/kg. Also, CVC appeared to be more effective when given BID compared to QD, as the reduction in recruitment observed at 20 mg/kg BID (10 mg/kg/dose) was greater than the one observed at 20 mg/kg QD. Compared to the thioglycollate control (Group 2), the decreases in total leukocytes were 0.8, 22.2, 57.6 and 14.2% for Groups 3 to 6, respectively, while the decreases in monocytes were 5.7, 45.2, 76.5 and 26.0% for Groups 3 to 6, respectively. Dexamethasone decreased the total leukocyte counts by 26.6% and the monocytes by 38.1%. The data is presented in Figures 7-10.

Blood Cell Evaluation: There were no clear changes observed in circulating blood cell counts. Slight differences were observed in group means for total leukocytes and monocytes; however, the differences were considered related to individual variations and total leukocytes were within historical ranges for this strain of mice (280 to 3870 cells/µL). The group mean summary is presented in Table 12 and the individual data in Figure 7.

PK Analysis: Following BID dosing, plasma levels of CVC at the approximate trough (14 hours postdose) increased in a dose-related manner. At 20 mg/kg/day, lower plasma levels were seen with QD dosing as compared to BID dosing. The data is summarized in Table 12.

**Table 12- Circulating Blood Cell Counts (mean ± standard error)**

| **Group** | **Treatment** | **Total Leukocytes (cells/µL)** | **Monocytes/Macrophages (cells/µL)** |
|---|---|---|---|
| 1 | **Non-disease control** | 568 ± 85 | 4.96 ± 1.46 |
| 2 | **TG - Control BID** | 1043 ± 351 | 10.26 ± 6.94 |
| 3 | **TG - CVC BID 5 mg/kg/day** | 697 ± 119 | 2.67 ± 0.35 |
| 4 | **TG - CVC BID 20 mg/kg/day** | 1218 ± 298 | 9.41 ± 2.54 |
| 5 | **TG - CVC BID 100 mg/kg/day** | 1367 ± 392 | 8.10 ± 3.35 |
| 6 | **TG - CVC QD 20 mg/kg/day** | 1035 ± 183 | 4.04 ± 0.98 |
| 7 | **TG - Dexamethasone - QD 1 mg/kg/day** | 1172 ± 361 | 16.57 ± 5.91 |

Animals were dosed as outlined Table 13.

**Table 13 - Experimental Design**

| Group | Group treatment | Total Dose (mg/kg/day) | Dose (mg/kg/dose) | Dose volume (mL/kg) | Formulation conc. (mg/mL) | No. of Males |
|---|---|---|---|---|---|---|
| 1 | Non-disease control | 0 | 0 | 10 | 0 | 6 |
| 2 | TG - Control BID^{a} | 0 | 0 (BID) | 10 | 0 | 8 |
| 3 | TG - CVC BID | 5 | 2.5 (BID) | 10 | 0.25 | 6 |
| 4 | TG - CVC BID | 20 | 10 (BID) | 10 | 1 | 6 |
| 5 | TG - CVC BID | 100 | 50 (BID) | 10 | 5 | 6 |
| 6 | TG - CVC QD | 20 | 20 | 10 | 2 | 6 |
| 7 | TG - Dexamethasone -QD | 1 | 1 | 10 | 0.1 | 6 |

| | | | | | | |
|---|---|---|---|---|---|---|
| TG = Thioglycollate; ^{a} Control animals received the vehicle for CVC (0.5% (w/v) methylcellulose. 1% Tween® 80 (pH ∼ 1.3) in DI Water | | | | | | |

Animals received the test article by oral gavage from Days 1 to 5. The twice daily (BID) dosing in Groups 2 to 5 was separated by approximately 12 hours. Blood samples were collected on Day 6 for assessment of plasma levels. Samples were collected by intracardiac puncture or from the vena cava, placed in a K2EDTA tube, and processed to plasma. Samples were stored frozen at -70 to -90° until analyzed. Due to the sacrifice time (48 hours after thioglycollate administration) being dictated by the primary endpoint, samples were collected approximately 14 hours postdose for Groups 2-5 and 26 hours postdose for the remaining groups. No samples were collected for animals 105, 201, 303 and 503.

Levels of CVC in plasma were determined by KCAS (Shawnee, KS) using a previously validated LC/MS/MS monkey plasma method (50 µL assay, range 10.0 - 1920 ng/mL).

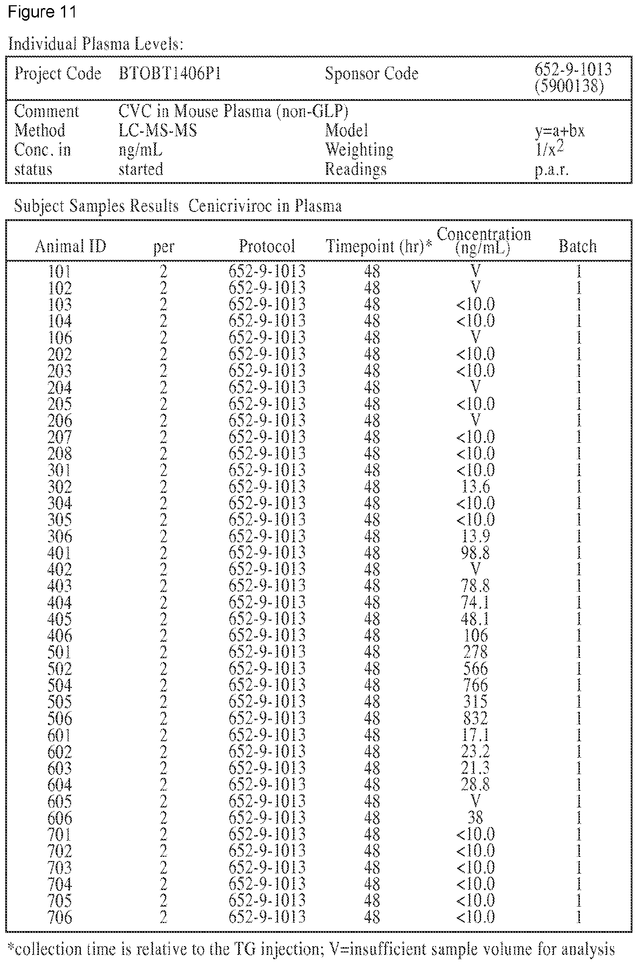

Table 14 provides mean plasma levels for each dose group that received CVC. Individual values for all dose groups are provided in Figure 11. No detectable CVC was noted in Groups 1, 2 and 7.

**Table 14 - Plasma levels (mean and standard deviation) of CVC in mice on Day 6**

| **Group** | **Dose** | **Time postdose*** | **CVC concentration (ng/mL)** | **N** |
|---|---|---|---|---|
| 3 | 5 mg/kg/day (BID) | ∼14 hours | 13.5** | 2 |
| 4 | 20 mg/kg/day (BID) | ∼14 hours | 81.2 ± 22.8 | 5 |
| 5 | 100 mg/kg/day (BID) | ∼14 hours | 551 ± 253 | 5 |
| 6 | 20 mg/kg/day (QD) | ∼26 hours | 25.7 ± 8.1 | 5 |

| | | | | |
|---|---|---|---|---|
| *nominal times; actual collection times were 14.5 hours (Group 3), 15 hours (Group 4), 15.5 hours (group 5) and 28 hours (Group 6) ** 3 of 5 samples below LLOQ (10 ng/mL) | | | | |

Following BID dosing, plasma levels of CVC at the approximate trough (∼14-16 hours postdose) increased in a dose-related manner. At 20 mg/kg/day, lower plasma levels were seen with QD dosing as compared to BID dosing.

Conclusion: Following CVC administration in the murine thioglycollate peritonitis model, a clear doserelated decrease was observed in peritoneal inflammatory cell recruitment, both on total leukocyte and mononuclear populations. There was a dose related increase in plasma exposures of CVC at the three dose levels. CVC appeared to be more efficacious when given BID compared to QD, in line with the higher plasma concentrations achieved with BID dosing and the known short half-life in mice (∼2 hours).

### Example 13- CCR2+ infiltrating monocytes promote acetaminophen-induced acute liver injury-therapeutic implications of inhibiting CCR2 and CCL2

Background and Aims: Liver injury following acetaminophen (APAP) intoxication is one of the leading courses of acute liver failure (ALF). APAP causes necrosis of hepatocytes followed by an activation of resident immune cells like Kupffer cells (KC), release of various chemokines (e.g., CCL2) and immune cell infiltration (e.g., monocytes). CCR2+ monocytes promote APAP-induced injury and investigated the therapeutic potential of pharmacologically blocking either CCR2 or CCL2.

Methods: C57BL/6J (WT) and Ccr2-/- mice were subjected to ALF by iv. injection of APAP (250 mg/kg body weight). Liver injury and immune cell phenotypes were analyzed in Ccr2-/- and WT mice, as well as in WT mice treated with mNOX-E36 s.c., a potent CCL2 inhibitor, or with the oral CCR2/CCR5 antagonist cenicriviroc (CVC).

Results: Ccr2-/- mice showed significantly reduced liver injury compared to WT mice 12h after APAP injection as determined by histology and reduced ALT values (p<0.05, Figure 62). Flow cytometry analyses revealed significantly reduced numbers of pro-inflammatory Ly6C+ monocyte-derived macrophages in livers of Ccr2-/- mice, whereas the numbers of neutrophils or other immune cell subsets remained similar to WT mice. While hepatic IL1β, TNF-α and CCL2 were similarly increased in both WT and Ccr2-/- mice, IL10 was higher in livers from Ccr2-/- mice (p<0.05), alongside differential marker expression (CD1d, CD68) on hepatic macrophages. Both pharmacological inhibitors, mNOX-E36 or CVC, were capable of significantly reducing monocyte accumulation in APAP-injured livers, resulting in significant protection from liver damage (ALT levels, p<0.05 for mNOX and p<0.01 for CVC).

Conclusions: Targeting detrimental actions of pro-inflammatory monocytes by inhibiting either the chemokine receptor CCR2 or its ligand CCL2 (MCP-1) is a promising therapeutic option to restrict liver injury following an acetaminophen overdose.

### Example 14: Dual CCR2/CCR5 Antagonist Cenicriviroc Leads to Potent and Significant Reduction in Proinflammatory CCR2+ Monocyte Infiltration in Experimental Acute Liver Injury

**Aim of the study:** Acute liver failure (ALF) is a life-threatening condition with rapid deterioration of hepatic function and limited therapeutic options. In mouse models, liver injury by toxic agents like carbon tetrachloride (CCl4) or acetaminophen (APAP) leads to rapid pro-inflammatory monocyte infiltration into the liver via the CCR2-CCL2 (a.k.a. MCP-1) chemokine pathway. Cenicriviroc (CVC) is an oral, once-daily CCR2/CCR5 antagonist currently evaluated in a Phase- 2b clinical trial in adults with NASH and liver fibrosis. CVC was evaluated for inhibiting monocyte infiltration in CCl4 and APAP-induced acute liver injury *in vivo.*

**Methods:** C57BL/6J (WT) and CCR2-deficient mice were subjected to ALF either by CCl4 (0.6 ml/kg IP) or APAP (250 mg/kg IV). In both models, mice received either CVC (100 mg/kg) or vehicle by oral gavage. Liver injury and immune cell phenotypes were analyzed. For mechanistic studies, monocyte-derived macrophage subsets and resident macrophages (Kupffer cells) were sorted by FACS from injured livers and subjected to array-based Nanostring gene expression analysis.

**Results:** Both CCl4 and APAP led to a rapid and massive accumulation of Ly6C+, monocyte-derived macrophages in injured livers, dependent on the chemokine receptor CCR2. Oral administration of CVC significantly reduced pro-inflammatory Ly6C+ monocytes in blood (p<0.01) and monocyte-derived macrophages in the liver for both CCl4 and APAP-induced acute liver injury. CVC treatment decreased Ly6C+ monocyte-derived macrophages in livers from 5.49% ± 0.49 (of liver leukocytes) to 0.95% ± 0.14 at 36h after CCl4 and from 6.01% ± 0.66 to 0.95% ± 0.11 at 12h after APAP (p<0.001 for both models, 83-84% reduction). Inhibition of infiltrating monocytes by CVC was associated with a significant protection from APAP-induced liver injury by reduction in ALT from 4365 U/L ± 951 to 1088 U/L ± 486 (p<0.01, 75% reduction) and in necrotic area fraction from 27.9% ± 4.05 to 10.9% ± 3.50 (p<0.01, 61% reduction). Additionally, Nanostring gene analysis revealed upregulated expression of chemokines, chemokine receptors and tolllike receptors in Ly6C+ CCR2+ monocyte-derived macrophages in comparison to their Ly6C- CCR2- counterparts in acute liver injury. In turn, adoptive transfer of bone marrow-derived monocytes aggravated APAP-induced liver injury, further corroborating the pro-inflammatory phenotype of CCR2+ monocytes in ALF.

**Conclusions:** CVC is a potent inhibitor of infiltration of pro-inflammatory monocytes into the liver in models of acute liver injury. Additionally, CVC may be considered as a promising therapeutic option to restrict liver injury following an acetaminophen overdose.

*Example 15: CCR2*+ *infiltrating monocytes promote acetaminophen-induced acute liver injury* - *Therapeutic implications of inhibiting CCR2 and CCL2***Background and Aims:** Liver injury following acetaminophen (APAP) intoxication is one of the leading courses of acute liver failure (ALF). APAP causes necrosis of hepatocytes followed by an activation of resident immune cells like Kupffer cells (KC), release of various chemokines (e.g., CCL2) and immune cell infiltration (e.g., monocytes). We hypothesized that CCR2+ monocytes promote APAP-induced injury and investigated the therapeutic potential of pharmacologically blocking either CCR2 or CCL2.

**Methods:** C57BL/6J (WT) and Ccr2-/- mice were subjected to ALF by iv. injection of APAP (250 mg/kg body weight). Liver injury and immune cell phenotypes were analyzed in Ccr2-/- and WT mice, as well as in WT mice treated with mNOX-E36 s.c., a potent CCL2 inhibitor, or with the oral CCR2/CCR5 antagonist cenicriviroc (CVC). The human counterparts of both agents are currently tested in phase II trials for different indications (diabetic nephropathy or NASH, respectively).

**Results:** Ccr2-/- mice showed significantly reduced liver injury compared to WT mice 12h after APAP injection as determined by histology and reduced ALT values (p<0.05, Figure 63). Flow cytometry analyses revealed significantly reduced numbers of pro-inflammatory Ly6C+ monocyte-derived macrophages in livers of Ccr2-/- mice, whereas the numbers of neutrophils or other immune cell subsets remained similar to WT mice. While hepatic IL1β, TNF-α and CCL2 were similarly increased in both WT and Ccr2-/- mice, IL10 was higher in livers from Ccr2-/- mice (p<0.05), alongside differential marker expression (CD1d, CD68) on hepatic macrophages. Both pharmacological inhibitors, mNOX-E36 or CVC, were capable of significantly reducing monocyte accumulation in APAP-injured livers, resulting in significant protection from liver damage (ALT levels, p<0.05 for mNOX and p<0.01 for CVC).

**Conclusions:** Targeting detrimental actions of pro-inflammatory monocytes by inhibiting either the chemokine receptor CCR2 or its ligand CCL2 (MCP-1) is a promising therapeutic option to restrict liver injury following an acetaminophen overdose.

### References:

1. Saiman Y, Friedman SL. The role of chemokines in acute liver injury. 2012;3:213.
2. Zimmermann HW, Tacke F. Modification of chemokine pathways and immune cell infiltration as a novel therapeutic approach in liver inflammation and fibrosis. Inflamm Allergy Drug Targets. 2011;10:509-536.
3. Seki E, De Minicis S, Gwak GY, Kluwe J, Inokuchi S, Bursill CA, Llovet JM, Brenner DA, Schwabe RF. CCR1 and CCR5 promote hepatic fibrosis in mice. J Clin Invest. 2009;119:1858-1870.
4. Seki E, de Minicis S, Inokuchi S, Taura K, Miyai K, van Rooijen N, Schwabe RF, Brenner DA. CCR2 promotes hepatic fibrosis in mice. Hepatology. 2009;50:185-197.
5. Miura K, Yang L, van Rooijen N, Ohnishi H, Seki E. Hepatic recruitment of macrophages promotes nonalcoholic steatohepatitis through CCR2. Am J Physiol Gastrointest Liver Physiol. 2012;302:G1310-1331.
6. Mitchell C, Couton D, Couty JP, Anson M, Crain AM, Bizet V, Rénia L, Pol S, Mallet V, Gilgenkrantz H. Dual role of CCR2 in the constitution and the resolution of liver fibrosis in mice. Am J Pathol. 2009;174:1766-1775.
7. Xia Y, Entman ML, Wang Y. CCR2 regulates the uptake of bone marrow-derived fibroblasts in renal fibrosis. PLoS ONE 2013; 8(10): e77493. doi:10.1371/journal.pone.0077493
8. Karlmark KR, Wasmuth HE, Trautwein C, Tacke F. Chemokine-directed immune cell infiltration in acute and chronic liver disease. Expert Review Gastroenterology Hepatology. 2008; 2: 233-242
9. Vielhauer V, Anders H-J, Mack M, Cihak J, Strutz F, Stangassinger M, Luckow B, Gröne H-J, Schlondorff D. Obstructive nephropathy in the mouse: Progressive fibrosis correlates with tubulointerstitial chemokine expression and accumulation of CC chemokine receptor 2- and 5-positive leukocytes. J Am Soc Nephrol 2001;12:1173-1187.
10. Segerer S, Mack M, Regele H, Kerjaschki D, Schlondorff D. Expression of the C-C chemokine receptor 5 in human kidney disease. Kidney Int 1999; 56:52-64.
11. Wai C-T, Greenson J, Fontana R, Kalbfleisch J, Marrero J, Conjeevaram H, Lok A. A simple noninvasive index can predict both significant fibrosis and cirrhosis in patients with chronic hepatitis C. Hepatology 2003;38:518-526.
12. Vallet-Pichard A, Mallet V, Nalpas B, Verkarre V, Nalpas A, Dhalluin-Venier V, Fontaine H, Pol S. FIB-4: an inexpensive and accurate marker of fibrosis in HCV infection. Comparison with liver biopsy and FibroTest. Hepatology 2007;46:32-36.
13. Sandler NG, Wand H, Roque A, et al. Plasma levels of soluble CD14 independently predict mortality in HIV infection. JID 2011;203:780-790.
14. Brenchley J, Price DA, Schacker TW, Asher TE, Silvestri G, Rao S, Kazzaz Z, Bornstein E, Lambotte O, Altmann D, Blazar BR, Rodriguez B, Teixeira-Johnson L, Landay A, Martin JN, Hecht FM, Picker LJ, Lederman MM, Deeks SG, Douek DC. Microbial translocation is a cause of systemic immune activation in chronic HIV infection.Nature Medicine 2006;12:1365-1371.
15. Vajro P, Paolella G, Fasano A. Microbiota and Gut-Liver Axis: Their Influences on Obesity and Obesity-Related Liver Disease JPGN 2013;56: 461-468.
16. Roh YS, Seki E. Toll-like receptors in alcoholic liver disease, non-alcoholic steatohepatitis and carcinogenesis Journal of Gastroenterology and Hepatology 2013; 28: 38-42.
17. Ilan Y. Leaky gut and the liver: A role for bacterial translocation in nonalcoholic steatohepatitis. World J Gastroenterol 2012 June 7; 18: 2609-2618.
18. Petrasek J, Mandrekar P, Szabo G. Toll-Like Receptors in the Pathogenesis of Alcoholic Liver Disease. Gastroenterology Research and Practice 2010, Article ID 710381, 12 pages. doi:10.1155/2010/710381.
19. Sandler N, Koh C, Roque A, Eccleston J, Siegel R, Demino M, Kleiner D, Deeks S, Liang T-J, Heller T, Douek D. Host Response to Translocated Microbial Products Predicts Outcomes of Patients With HBV or HCV Infection. Gastroenterology 2011;141:1220-1230. doi:10.1053/j.gastro.2011.06.063.
20. Wiest R, Lawson M, Geuking M. Pathological bacterial translocation in liver cirrhosis. J Hepatology 2014; 60(1): 197-209.
21. Shanab AA, Scully P, Crosbie O, Buckley M, O'Mahony L, Shanahan F, Gazareen S, Murphy E, Quigley EM. Small intestinal bacterial overgrowth in nonalcoholic steatohepatitis: association with toll-like receptor 4 expression and plasma levels of interleukin 8. Dig Dis Sci 2011; 56: 1524-1534
22. Henao-Mejia J, Elinav E, Jin C, Hao L, Mehal WZ, Strowig T, Thaiss CA, Kau AL, Eisenbarth SC, Jurczak MJ, Camporez J-P, Shulman GI, Gordon JI, Hoffman HM; Flavell RA. Inflammasome-mediated dysbiosis regulates progression of NAFLD and obesity. Nature 2012; 492: 179-185. doi:10.1038/nature10809.
23. Klibanov, Olga M.; Williams, Shannon H.; Iler, Cameron A (2010). "Cenicriviroc, an orally active CCR5 antagonist for the potential treatment of HIV infection". Current Opinion in Investigational Drugs 11 (8): 940-950.
**24.** Kleiner DE. et al., Hepatology, Design and validation of a histological scoring system for nonalcoholic fatty liver disease; 2005;41:1313-1321.
25. Fujii H et al. J. Atheroscler. Thromb. 2009;16:893.
26. Rangwala F et al. J. Pathol. 2011; 224:401.
27. Seki et al. CCR1 and CCR5 promote hepatic fibrosis in mice. J. Clin. Invest. 2009; 119(7):1858-1870.
28. Xu et al. Liver fibrosis: mechanisms of immune-mediated liver injury. Cellular & Mol. Immunol.; 2012; 9:296-301.
29. Karlmark et al, Expert Rev. Gastroenterol. Hepatol. 2(2), 233-242 (2008).
30. Mitchell C, et al. Dual role of CCR2 in the constitution and the resolution of liver fibrosis in mice. Am J Pathol. 2009;174:1766-1775.
31. Berres M-L, et al. Antagonism of the chemokine Ccl5 ameliorates experimental liver fibrosis in mice. Journal of Clin Invest. November 2010; 120(11): 4129-4140
32. Benyon, RC and MJ Arthur, Extracellular matrix degradation and the role of hepatic stellate cells.Semin Liver Dis. 2001 Aug;21(3):373-84.
33. Sheth SG, Chopra S. Natural history and management of nonalcoholic fatty liver disease in adults, UpToDate; 2014
34. Chalasani N, Younossi Z, Lavine JE, Diehl AM, Brunt EM, Cusi K, et al. The diagnosis and management of non-alcoholic fatty liver disease: Practice Guideline by the American Association for the Study of Liver Diseases, American College of Gastroenterology, and the American Gastroenterological Association. Hepatology 2012;55:2005-23.
35. McCullough AJ. The clinical features, diagnosis and natural history of nonalcoholic fatty liver disease. Clin Liver Dis 2004;8(3):521-33.
36. Loomba R, Sirlin CB, Schwimmer JB, Lavine JE. Advances in pediatric nonalcoholic fatty liver disease. Hepatology 2009;50(4):1282-93.
37. Torres DM, Williams CD, Harrison SA. Features, diagnosis, and treatment of nonalcoholic fatty liver disease. Clin Gastroenterol Hepatol 2012;10(8):837-58.
38. Schwenger KJ, Allard JP. Clinical approaches to non-alcoholic fatty liver disease. World J Gastroenterol 2014;20(7):1712-23.
39. Koo SH. Nonalcoholic fatty liver disease: molecular mechanisms for the hepatic steatosis. Clin Mol Hepatol 2013;19(3):210-5.
40. Attar BM, Van Thiel DH. Current concepts and management approaches in nonalcoholic fatty liver disease. ScientificWorld Journal 2013;2013:481893
41. Basaranoglu M, Basaranoglu G, Sentürk H. From fatty liver to fibrosis: a tale of "second hit". World J Gastroenterol 2013;19(8):1158-65.
42. Cohen JC, Horton JD, Hobbs HH. Human fatty liver disease: old questions and new insights. Science 2011;332(6037):1519-23.
43. Matteoni CA, Younossi ZA, Gramlich T, et al. Nonalcoholic fatty liver disease: A spectrum of clinical and pathological severity. Gastroenterology 1999;116(6):1413-1419
44. World Gastroenterology Organisation Global Guidelines. Nonalcoholic Fatty liver Disease and Nonalcoholic Steatohepatitis. June 2012.
45. Ahmed MH, Abu EO, Byrne CD. Non-Alcoholic Fatty Liver Disease (NAFLD): new challenge for general practitioners and important burden for health authorities? Prim Care Diabetes. 2010;4:129-37.
46. Vernon G, Baranova A, Younossi ZM. Systematic review: the epidemiology and natural history of non-alcoholic fatty liver disease and nonalcoholic steatohepatitis in adults. Aliment Pharmacol Ther 2011;34:274-85.
47. The Gastroenterological Society of Australia/Australian Liver Association January 2013. http://static.squarespace.com/static/50ff0804e4b007d5a9abe0a5/t/53321aaee4b09f967eb0c7e5/ 1395792558684/gesa2013_revised%5B1%5D.pdf
48. Dixon JB, Bhathal PS, O'Brien PE. Nonalcoholic fatty liver disease: predictors of nonalcoholic steatohepatitis and liver fibrosis in the severely obese. Gastroenterol. 2001;121:91-100.
49. Williams CD, Stenger J, Asike MI, Torres DM, Shaw J, Contreras M, et al. Prevalence of nonalcoholic fatty liver disease and nonalcoholic steatohepatitis among a largely middle-aged population utilizing ultrasound and liver biopsy: a prospective study. Gastroenterology 2011;140:124-131.
50. Schattenberg JM, Schuppan D. Nonalcoholic steatohepatitis: the therapeutic challenge of a global epidemic. Curr Opin Lipidol 2011;22:479-88.
51. Bahrami H. Nonalcoholic fatty liver disease in developing countries. World J Gastroenterol 2005;11:3808-3809.
52. Tiniakos DG, Vos MB, Brunt EM. Nonalcoholic fatty liver disease: pathology and pathogenesis. Annu Rev Pathol 2010;5:145-71.
53. Vajro P, Paolella G, Fasano A. Microbiota and gut-liver axis: their influences on obesity and obesity-related liver disease. J Pediatr Gastroenterol Nutr 2013;56:461-8.
54. Roh YS, Seki E. Toll-like receptors in alcoholic liver disease, non-alcoholic steatohepatitis and carcinogenesis. J Gastroenterol Hepatol 2013;28 Suppl 1:38-42.
55. Ilan Y. Leaky gut and the liver: a role for bacterial translocation in nonalcoholic steatohepatitis. World J Gastroenterol 2012;18:2609-18.
56. Moschen AR, Kaser S, Tilg H. Non-alcoholic steatohepatitis: a microbiota-driven disease. Trends Endocrinol Metab 2013;24:537-45.
57. Sanyal AJ, Campbell-Sargent C, Mirshahi F, Rizzo WB, Contos MJ, Sterling RK, et al. Nonalcoholic steatohepatitis: association of insulin resistance and mitochondrial abnormalities. Gastroenterology 2001;120:1183-92.
58. McClain CJ, Mokshagundam SP, Barve SS, Song Z, Hill DB, Chen T, et al. Mechanisms of non-alcoholic steatohepatitis. Alcohol 2004;34:67-79.
59. Day CP, Saksena S. Non-alcoholic steatohepatitis: definitions and pathogenesis. J Gastroenterol Hepatol. 2002;17 Suppl 3:S377-S84.
60. Marra F, Gastaldelli A, Svegliati Baroni G, Tell G, Tiribelli C. Molecular basis and mechanisms of progression of non-alcoholic steatohepatitis. Trends Mol Med. 2008;14:72-81
61. Charlton MR, Burns JM, Pederson RA, Watt KD, Heimbach JK, Dierkhising RA. Frequency and outcomes of liver transplantation for nonalcoholic steatohepatitis in the United States. Gastroenterol. 2011;141:1249-53.
62. Vatche G. Agopian et al. Liver Transplantation for Nonalcoholic Steatohepatitis. Annals of Surgery 2012; 256(4); 624-633.
63. McCullough AJ. Epidemiology of the metabolic syndrome in the USA. J Dig Dis. 2011;12:333-40.

## Claims

1. A pharmaceutical composition comprising cenicriviroc or a salt or solvate thereof and fumaric acid for use in the treatment of acute liver injury in a subject in need thereof, wherein the acute liver injury is acetaminophen-induced, and/or toxin-induced liver injury and wherein the pharmaceutical composition is administered to a subject in need thereof in a therapeutically effective amount once per day or twice per day.

2. A pharmaceutical composition comprising cenicriviroc or a salt or solvate thereof and fumaric acid for use in the treatment of peritonitis in a subject in need thereof;
wherein the pharmaceutical composition is administered to the subject in a therapeutically effective amount twice per day.

3. The pharmaceutical composition for use according to claim 2, wherein the peritonitis is infected or non-infected.

4. The pharmaceutical composition for use according to claim 2, wherein the peritonitis is associated with a perforation of the gastrointestinal tract, leakage of fluid into the peritoneum, or a foreign body.

5. The pharmaceutical composition for use according to any of the preceding claims, wherein the pharmaceutical composition is coadministered with one or more additional active agents or treatments;
wherein the one or more additional active agents or treatments are selected from the group consisting of one or more antibiotic, glucocorticoid, corticosteroid, Pentoxifylline, phosphodiesterase inhibitor, anti-TNFa agent, anti-oxidants and n-acetylcysteine (acetylcysteine; NAC).

6. The pharmaceutical composition for use according to claim 5, wherein the one or more antibiotics is selected from the group consisting of penicillins, cephalosporins, macrolides, fluoroquinolones, sulfonamides, tetracyclines, and aminoglycosides or a combination thereof.

7. The pharmaceutical composition for use according to claim 5, wherein the additional active agent or treatment is NAC and is administered either intravenously or orally.

## Patentansprüche

1. Pharmazeutische Zusammensetzung, umfassend Cenicriviroc oder ein Salz oder Solvat davon und Fumarsäure zur Verwendung in der Behandlung von akuten Leberschäden in einem Subjekt, das dessen bedarf, wobei die akuten Leberschäden Acetaminophen-induzierte und/oder Toxin-induzierte Leberschäden sind, und wobei die pharmazeutische Zusammensetzung an ein Subjekt, das dessen bedarf, in einer therapeutisch wirksamen Menge einmal täglich oder zweimal täglich verabreicht wird.

2. Pharmazeutische Zusammensetzung, umfassend Cenicriviroc oder ein Salz oder Solvat davon und Fumarsäure zur Verwendung in der Behandlung von Peritonitis in einem Subjekt, das dessen bedarf;
wobei die pharmazeutische Zusammensetzung dem Subjekt in einer therapeutisch wirksamen Menge zweimal täglich verabreicht wird.

3. Pharmazeutische Zusammensetzung nach Anspruch 2, wobei die Peritonitis infiziert oder nicht infiziert ist.

4. Pharmazeutische Zusammensetzung zur Verwendung nach Anspruch 2, wobei die Peritonitis mit einer Perforation des Magen-Darm-Trakts, Austreten von Flüssigkeit in das Peritoneum oder einem Fremdkörper assoziiert ist.

5. Pharmazeutische Zusammensetzung zur Verwendung nach einem der vorhergehenden Ansprüche, wobei die pharmazeutische Zusammensetzung mit einem oder mehreren zusätzlichen Wirkstoffen oder Behandlungen zusammen verabreicht wird;
wobei der/die eine oder die mehreren zusätzlichen Wirkstoffe oder Behandlungen ausgewählt sind aus der Gruppe bestehend aus einem oder mehreren Antibiotika, Glukokortikoiden, Kortikosteroiden, Pentoxifyllin, Phosphodiesterase-Hemmer, anti-TNFa-Mittel, Antioxidantien und n-Acetylcystein (Acetylcystein; NAC).

6. Pharmazeutische Zusammensetzung zur Verwendung nach Anspruch 5, wobei das eine oder die mehreren Antibiotika ausgewählt ist aus der Gruppe bestehend aus Penicillinen, Cephalosporinen, Makroliden, Fluorchinolonen, Sulfonamiden, Tetracyclinen und Aminoglykosiden oder einer Kombination davon.

7. Pharmazeutische Zusammensetzung zur Verwendung nach Anspruch 5, wobei der/die zusätzliche Wirkstoff oder Behandlung NAC ist und entweder intravenös oder oral verabreicht wird.

## Revendications

1. Composition pharmaceutique, comprenant du cénicriviroc ou un sel ou solvate de celui-ci et de l'acide fumarique, pour l'utilisation dans le traitement de lésion hépatique aiguë chez un sujet en ayant besoin, dans laquelle la lésion hépatique aiguë est une lésion hépatique induite par acétaminophène, et/ou induite par toxine et dans laquelle la composition pharmaceutique est administrée à un sujet en ayant besoin en une quantité thérapeutiquement efficace une fois par jour ou deux fois par jour.

2. Composition pharmaceutique, comprenant du cénicriviroc ou un sel ou solvate de celui-ci et de l'acide fumarique, pour l'utilisation dans le traitement de péritonite chez un sujet en ayant besoin ;
dans laquelle la composition pharmaceutique est administrée au sujet en une quantité thérapeutiquement efficace deux fois par jour.

3. Composition pharmaceutique pour l'utilisation selon la revendication 2, dans laquelle la péritonite est infectée ou non infectée.

4. Composition pharmaceutique pour l'utilisation selon la revendication 2, dans laquelle la péritonite est associée à une perforation du tractus gastrointestinale, une fuite de fluide dans le péritoine, ou un corps étranger.

5. Composition pharmaceutique pour l'utilisation selon l'une quelconque des revendications précédentes, dans laquelle la composition pharmaceutique est coadministrée avec un ou plusieurs agents ou traitements actifs supplémentaires ;
dans laquelle les un ou plusieurs agents ou traitements actifs supplémentaires sont sélectionnés parmi le groupe constitué d'un ou de plusieurs antibiotique, glucocorticoïde, corticostéroïde, pentoxifylline, inhibiteur de phosphodiestérase, agent anti-TNFα, anti-oxydants et n-acétylcystéine (acétylcystéine ; NAC).

6. Composition pharmaceutique pour l'utilisation selon la revendication 5, dans laquelle les un ou plusieurs antibiotiques est sélectionné parmi le groupe constitué de pénicillines, de céphalosporines, de macrolides, de fluoroquinolones, de sulfonamides, de tétracyclines, et d'aminoglycosides ou d'une association de ceux-ci.

7. Composition pharmaceutique pour l'utilisation selon la revendication 5, dans laquelle l'agent ou traitement actif supplémentaire est NAC et est administré soit de façon intraveineuse soit de façon orale.
